# EUROPEAN PATENT SPECIFICATION

(11) **EP 2 646 003 B1**
(45) Date of publication and mention of the grant of the patent: **03.06.2020**
(21) Application number: 11845725.8
(22) Date of filing: 02.12.2011
(51) Int. Cl.: A61K 9/00, A61K 9/16, A61K 9/20, A61K 31/137, A61K 31/167, A61K 31/351, A61K 31/4453, A61K 31/485, A61K 31/53, A61K 31/5513

(54) **RAPIDLY DISPERSING GRANULES, ORALLY DISINTEGRATING TABLETS AND METHODS**
SCHNELL DISPERGIERENDES GRANULAT, IM MUND ZERFALLENDE TABLETTEN UND VERFAHREN DAFÜR
GRANULES À DISPERSION RAPIDE, COMPRIMÉS SE DÉLITANT ORALEMENT ET PROCÉDÉS AFFÉRENTS

(30) Priority: 02.12.2010 US 419114 P
(43) Date of publication of application: 09.10.2013
(73) Proprietor: Adare Pharmaceuticals, Inc., Lawrenceville, NJ 08648 (US)
(72) Inventor: VENKATESH, Gopi M., Vandalia Ohio 45377 (US); SWAMINATHAN, Vijaya, Beavercreek Ohio 45431 (US); LAI, Jin-Wang, Springboro Ohio 45066 (US); CLEVENGER, James M., Vandalia Ohio 45377 (US)
(74) Representative: Cooley (UK) LLP
(86) International application number: PCT/US2011/063172
(87) International publication number: WO 2012/075455

(56) References cited:
- US-A1- 2005 079 138
- US-A1- 2009 092 672
- US-A1- 2009 169 620
- US-B1- 6 569 455
- ARTHUR H KIBBE ED - KIBBE A H (ED): "Starch, Pregelatinized", 1 January 2000 (2000-01-01), HANDBOOK OF PHARMACEUTICAL EXCIPIENTS, AMERICAN PHARMACEUTICAL ASSOC. [U.A.], WASHINGTON, DC; US, PAGE(S) 528 - 529, XP002471274, ISBN: 978-0-85369-381-9 * the whole document * * Sections 4, 6, 7 *
- PARIND MAHENDRAKUMAR DESAI ET AL: "Review of Disintegrants and the Disintegration Phenomena", JOURNAL OF PHARMACEUTICAL SCIENCES, vol. 105, no. 9, 1 September 2016 (2016-09-01), pages 2545-2555, XP055607494, US ISSN: 0022-3549, DOI: 10.1016/j.xphs.2015.12.019

## Description

### FIELD OF THE INVENTION

This invention relates to rapidly dispersing microgranules which can be incorporated into an orally disintegrating tablet (ODT) that disintegrates in the oral cavity of a mammal, without the need of water or other fluids.

### BACKGROUND OF THE INVENTION

Non-adherence to dosing regimens is a major medical problem in America costing billions of dollars. Taking a medication isn't always as simple as swallowing a pill. Taking medications exactly as prescribed and following appropriate lifestyle recommendations are highly beneficial and may reduce the impact of side effects. Medication non-compliance (non-adherence), the failure to take drugs on time in the dosages prescribed, is as dangerous and costly as many illnesses. Studies have shown that non-compliance causes 125,000 deaths annually in the US, leads to 10 to 25 percent of hospital and nursing home admissions, and is becoming an international epidemic. In addition, patient adherence or compliance to dosing regimens has become a major concern costing millions of dollars. Complicated regimen (e.g., too many medications, too frequent dosing), physical difficulty in complying (e.g., opening medicine containers, handling small tablets, swallowing difficulties (e.g., about 30% of the general population), timely accessibility of drinks), willful refusal including "medication cheeking" for later discarding, real or perceived side-effects and lack of effectiveness, unattractive formulations (e.g., unpleasant taste or odor) are often cited as factors responsible for non-compliance. It is often observed that some patients with diseases such as schizophrenia, bipolar disorders are often disorganized or have memory problems (cognitive dysfunction) and fail to take medications regularly.

There are various types of pharmaceutical dosage forms for oral administration: tablets, capsules, sachets, powders for reconstitution into suspensions, syrups and so on. However, such dosage forms have several problems. In case of tablets and capsules, for example, it may be hard to administer medication to aged persons or children who are unwilling or experience difficulty swallowing due to dysphagia. Suspensions, syrups, sachets, etc. containing medicaments are often too bitter to be consumed orally due to unpleasant mouthfeel. Further, 'people on the move' due to their lifestyle or migraine patients when in need may not have easy access to water or drinks.

On the other hand, solid pharmaceutical compositions comprising microparticles of the drug that are well taste-masked and/or coated with functional polymers to impart sustained, delayed or timed, pulsatile release properties, which rapidly disintegrate in the buccal cavity forming a smooth (non-gritty), easy-to-swallow suspension with non-gritty mouthfeel, and which, upon being swallowed without the need for water or experiencing any aftertaste, exhibit target *in vitro* drug release profiles that are very much needed to provide convenience of oral administration and to improve patient adherence or compliance to dosing regimens.

US Patent 4,134,943 relates to a process for the production of porous tablets having an excellent disintegrating property, which comprises mixing contents of the tablet with an inert solvent and freeze drying. Zydis® technology (US Patent 4,305,502; US 5,738,875), Lyoc technology (US Patent 4,616,047; US 5,843,347), and QuickSolv® technology (US Patent 5,215,756; US 5,298,261) allow removal of water from frozen blisters by sublimation/freeze-drying at low temperature, producing freeze dried sugar, lactose, maltodextrin, and/or gelatin matrix based rapidly dissolving tablets/wafers. The major disadvantages of the lyophilization technology include that it is expensive, provides for fragile products, is difficult to use with taste-masked drug particles, and provides a poor mouthfeel and stability under stressed conditions.

US Patents 5,039,540 and 5,079,018 relate to a method for the production of tablets with sufficient strength, by allowing the contents of the tablet to be contacted by an anhydrous organic liquid such as anhydrous ethanol at 0°C or lower until all of the water content is substantially removed from the composition. Each of these production processes requires complex production steps and additional equipment such as freeze dryer, specialized packaging equipment, and the like, thus entailing high production costs.

US Patent 5,720,974 relates to production methods for fast dissolving tablets with porous structure wherein tablets comprising a granulation of an active, a carbohydrate including a sugar, starch, lactose, or a sugar alcohol such as mannitol, having a particle size of 20 to 70 µm, granulated with 1 to 3% by weight of water, are produced by compressing the wet mass into tablets at low compression forces prior to drying, thereby requiring elaborate arrangements for handling individual tablets after compression until their bulk storage following drying of moist tablets.

Cima's OroSolv® technology (US Patent 5,178,878; US 6,155,423; US 6,311,462), DuraSolv® technology (US Patent 6,024,981), and OraVescent® technology (US Patent 6,200,604) relate to the production of compressed, rapidly disintegrating tablets comprising uncoated or taste-masked drug particles, and water soluble excipients. OraSolv® comprising an effervescent couple is very fragile, requiring an integrated tableting-packaging system. DuraSolv® comprising at least 60% w/w of powdered (non-direct compression) filler/excipient such as mannitol or compressible sugar, are hard tablets which are packaged in blisters or bottles. OraVescent® tablets comprising an effervescent component facilitate drug dissolution with a transient change in pH on contact with saliva. Both OroSolv® and OraVescent® technologies require expensive integrated tableting and packaging systems.

US Patent 5,464,632 relates to a method of manufacturing orally disintegrating tablets that disintegrate within 60 seconds in the buccal cavity without water, comprising an active substance (coated microcrystals or microgranules and a mixture of non-effervescent excipients including a disintegrant. These tablets are often gritty. WOWTAB® technology (US Patent 5,466,464 and US 5,576,014) relates to a method of producing of intrabuccally dissolving tablets wherein a saccharide having low moldability such as lactose or mannitol is granulated with an active and a saccharide having high moldability (e.g., sorbitol or maltitol), and the combined granulation after drying is blended with a lubricant and compressed into intrabuccally disintegrating tablets. Alternatively, the active ingredient may be separately granulated with a low moldability saccharide and subsequently compressed with a granulation of saccharides with high and low moldability into intrabuccally disintegrating tablets. SaTab technology (US Patent 6,316,026) utilizes a proprietary moistening and drying process to produce highly porous ODT formulations that disintegrate/dissolve in about 10 seconds by compressing into tablets a powder mixture comprising a sugar, a drug, and a binder under low compression pressure and passing the tablets through a specially designed equipment for moistening and drying.

According to US 20030215500 A1, orally disintegrating tablets comprising granules of a low moldability sugar alcohol such as mannitol or a saccharide such as lactose having a mean particle size of about 60µm and a super disintegrant such as crospovidone (e.g., Polyplasdone XL-10 from ISP) granulated with water in the presence or absence of an active ingredient, exhibit rapid disintegration in the buccal cavity while having poor mechanical strength. However, if a sugar alcohol or a saccharide having a median particle size of about 60 µm and a super disintegrant (e.g., crospovidone) in the presence or absence of an active ingredient such as domperidone, using a solution of a polymeric binder (e.g., povidone K-30 or hydroxypropylcellulose) or a high moldability sugar alcohol or saccharide (e.g., maltose) as the granulation fluid, the ODT tablets weighing 200 mg thus produced not only exhibited high tablet strength but also were shown to take 101-350 seconds to disintegrate in the oral cavity, depending on the binder used. If, on the other hand, the sugar alcohol and/or saccharide having a mean particle size of not more than 35 µm and a super disintegrant are granulated with water in the presence or absence of an active ingredient such as domperidone in accordance with the disclosures of US 20030215500 A1, in a high shear granulator followed by drying in a fluid bed dryer and compressing into tablets with internal or external lubrication, orally disintegrating tablets thus produced exhibit high mechanical strength, without compromising disintegration properties. In cases where a sugar alcohol (e.g., mannitol) or a saccharide (e.g., lactose) having a median particle size of not more than 30 µm and a super disintegrant (e.g., crospovidone) are granulated using purified water, the active ingredient is blended with the mannitol-crospovidone granules and compressed into ODT tablets.

US 20030215500 A1 does not relate to a method of taste-masking bitter drugs and/or the use of bitter drugs, especially at high doses (i.e., at > 30% by weight of the tablet) in orally disintegrating tablets. A large percentage of pharmacologically active drugs are bitter and require taste-masking as well as often high doses to be therapeutically effective.

According to US 20040122106 A1, orally disintegrating tablets comprising granules of a low moldability sugar alcohol such as mannitol having a mean particle size of not less than 30 µm, a super disintegrant, for example, crospovidone, and an active ingredient having an aqueous solubility of 1 mg/mL or higher granulated with water, exhibit rapid disintegration in the buccal cavity while having high mechanical strength. Many water soluble active ingredients are too bitter to be incorporated into ODTs without first taste-masking.

US20050232988 A1 relates to a method of preparing orally disintegrating tablets with no flow- and/or compression-related issues, comprising effectively taste-masked microgranules, the method comprising granulating a bitter drug such as ranitidine HCl or sumatriptan succinate and microencapsulating by solvent coacervation with ethylcellulose to form rapidly dispersing microgranules with a median particle size of about 160 µm. The method further comprises preparing a high shear granulation comprising mannitol having a median particle size of about 15 µm and crospovidone in a pilot scale GMX 25 - Glatt GPCG 5 system. The ODT tablets thus produced exhibit not only rapid disintegration on contact with saliva, but also exhibit a non-gritty mouthfeel and no aftertaste.

At the industrial scale operation (e.g., batch size in a GMX 600 high shear granulator-tray dryer (150-160 kg) or GMX 600-fluid bed dryer (Glatt GPCG 200): 300-320 kg), the following changes were required and/or observations were made:
- The use of an increased amount of the granulation fluid resulted in larger agglomerates which necessitated extensive milling of moist granulations before and after drying, and inclusion of a vacuum transfer system to charge into the dryer. This resulted in a significant increase in granulation time and hence cost of goods.
- In spite of the milling of moist granules to reduce oversized agglomerates upon drying, the process resulted in significant quantities of hard oversized agglomerates.
- Severe dry-milling of hard oversized agglomerates to achieve higher useable yields and reduced cost of goods resulted in irregularly shaped granules with sharp edges resulting in poor flow and compression properties.
- The use of conventional tray drying oven for drying of moist granulations instead of the Glatt GPCG 200 requiring the steps of evenly spreading wet-milled moist granulations on trays to about 2 inch in depth, milling of partially dried granules followed by drying for a loss on drying (LOD) of < 1% by weight, resulted in increased cost of goods.
- However, following extensive optimization, the performance properties of the rapidly dispersing microgranules produced at industrial scale manufacturing (batch size: 160-320 kg) are shown to be similar to that of the pilot/semi-industrial scale granulations produced in accordance with the disclosure of US 20030215500, when tableted alone or in combination with microencapsulated acetaminophen microparticles (i.e., at a drug load of 25% by weight of the tablet).

Trouble-free tablet manufacturing of ODT tablets comprising microencapsulated drug microparticles and industrial scale rapidly dispersing microgranules has been reported elsewhere (see ODT tablets comprising microencapsulated lamotrigine microcrystals (200 mg per tablet weighing 800 mg) in US 20090092672 A1); and microencapsulated acetaminophen microcrystals (500 mg per 1400 mg tablet); microencapsulated diphenhydramine HCl (DPH) microparticles (25 mg per 650 mg tablet) obtained by layering DPH onto 60-80 mesh (177-250 µm) sugar spheres are disclosed in US 20090155360; and ranitidine HCl microcrystals (168 mg per 1100 mg tablet) with a taste-masking dual membrane is disclosed in US 20090202630). However, the ODT formulations comprising taste-masked ranitidine HCl microcrystals, taste-masked acetaminophen microcrystals, and taste-masked DPH layered beads required the incorporation of a compression aid such as microcrystalline cellulose (e.g., Avicel PH101), respectively at 10%, 10%, and 20% by weight of the tablet, for trouble-free tablet manufacturing.

Furthermore, the ODT tablets (30 mg per 500 mg tablet) comprising industrial scale rapidly dispersing microgranules and fluid bed granulated temazepam microgranules comprising D-mannitol with a median particle size of about 15 µm, micronized temazepam, and crospovidone, required a polymeric binder at a low level (e.g., low viscosity hydroxypropylcellulose at 1-2% by weight for trouble-free tablet manufacturing at industrial scale (see US 20090169620)).

However, the ODT tablets comprising industrial scale rapidly dispersing microgranules and microencapsulated acetaminophen microparticles in combination with taste-masked acetaminophen-hydrocodone bitartrate microparticles, required not only a material flow enhancer (e.g., spray dried mannitol, Parteck® M 300 at 10% by weight) but also a compression aid such as microcrystalline cellulose for trouble-free long tableting runs.

The above discussed references do not relate to free flowing rapidly dispersing microgranules with a median particle size in the range of about 100 to about 300 µm (for example a range of about 150 to about 250 µm, or a range of about 200 to about 300 µm) comprising a sugar alcohol, a saccharide or a mixture thereof, and a super disintegrant. Furthermore, the references do not disclose or suggest that the rapidly dispersing microgranules would be suitable for blending with a pharmaceutically active agent that is optionally taste-masked and/or functional polymer coated drug microparticles to also provide for a pharmaceutical composition. In addition, the references do not disclose or suggest that such rapidly dispersing microgranules would be useful for compressing into orally disintegrating tablets that not only possess sufficiently high tablet hardness and low friability to maintain integrity during packaging into bottles and/or blisters, storage, transportation for commercial distribution and end use, but that also rapidly disintegrate on contact with saliva in the buccal cavity, forming a smooth, easy-to-swallow suspension with non-gritty mouthfeel or disintegrate within 30 seconds when tested with USP Method <701> for Disintegration Time, as required for orally disintegrating tablets per the FDA Guidance to Industry. Also not disclosed in the references are such rapidly dispersing microgranules that are free flowing and produced in a high useable yield. Likewise the references do not relate to a method of economically manufacturing such rapidly dispersing microgranules, or pharmaceutical compositions thereof. Furthermore, the references do not disclose or suggest a granulation method for providing a pharmaceutical composition as an ODT comprising an active pharmaceutical ingredient. Likewise, the references do not relate to a method of economically manufacturing such free-flowing, rapidly dispersing microgranules in a high useable yield, or pharmaceutical compositions thereof.

### SUMMARY OF THE INVENTION

The present invention, in one aspect, is directed to pharmaceutically acceptable rapidly dispersing microgranules having a median particle size in the range of 100 µm to 300 µm and comprising at least one sugar alcohol, saccharide, or a mixture thereof, at least one super disintegrant selected from the group consisting of crospovidone, croscarmellose sodium, sodium starch glycolate, low-substituted hydroxypropylcellulose and mixtures thereof, and pregelatinized starch in an amount of 0.5-3.0% by weight, wherein the rapidly dispersing microgranules do not comprise a pharmaceutically active agent.

In a second aspect, the invention provides a pharmaceutical dosage form, which is an orally disintegrating tablet, comprising: a) the rapidly dispersing microgranules of the first aspect of the invention; and b) a therapeutically effective amount of at least one active pharmaceutical ingredient.

The pharmaceutically acceptable rapidly dispersing microgranules of the first aspect may therefore be for blending with taste-masked microparticles comprising at least one pharmaceutically acceptable active to be incorporated into orally disintegrating tablets. In one embodiment, the tablet disintegrates in about 60 seconds in the oral cavity of a mammal, or about 50 seconds, or about 40 seconds, or about 30 seconds in the oral cavity of a mammal, without the need of water or other fluids. The present inventors developed rapidly dispersing microgranules which allow not only elimination of a wet milling step but also avoiding an extensive dry milling step. Furthermore, such rapidly dispersing microgranules are suitable for blending with a pharmaceutically active agent that is optionally taste-masked and/or controlled release coated microparticles to also provide for a pharmaceutical composition wherein the active agent in therapeutically effective amounts at a ratio of from 6:1 to 1:2 for compression into orally disintegrating tablets without requiring special production technology, equipment, and/or flow enhancing spray-dried excipients (e.g., Parteck M 200/M 300 which improves the flow of poorly flowing compression blends during tableting). The such rapidly dispersing microgranules according to the invention are free flowing and produced in a high useable yield.

The rapidly dispersing microgranules of the first aspect are suitable for producing orally disintegrating tablets having sufficient mechanical strength to resist attrition or chipping during packaging in PTP (press-through-package) or peel-off paper-backed blisters and HDPE bottles, storage, transportation, commercial distribution, and end-use and at the same time exhibiting rapid disintegration in the buccal cavity, in one embodiment, within 60 seconds with a smooth non-gritty mouthfeel, without chewing or the need for water or other fluids. In case of immediate release dosage forms, it is further anticipated the taste-masked drug particles exhibit rapid dissolution profiles similar to that of reference listed drug (RLD) to be bioequivalent to RLD to avoid expensive efficacy studies.

In a third aspect, the invention provides a method of manufacturing an orally disintegrating tablet comprising the following steps: (a) preparing active pharmaceutical ingredient microparticles; (b) coating the drug microparticles with one or more functional polymers to impart taste-masking and/or controlled release characteristics; (c) preparing a powder mixture comprising polymer coated drug microparticles having a median particle size in the range of 100 to 400 µm from step (b), free flowing, rapidly dispersing micro granules having a median particle size in the range of 100 to 300 µm of the first aspect, and other optional pharmaceutically acceptable excipients selected from a flavorant, sweetener, colorant, compression aid, and additional disintegrant; (d) compressing the powder mixture on a rotary tablet press using internal or external lubrication, wherein the orally disintegrating tablet rapidly disintegrates on contact with saliva in the buccal cavity into a smooth, non-gritty, easy-to-swallow suspension containing polymer coated drug microparticles or drug microparticles taste-masked by granulating with a sugar alcohol, super disintegrant and optionally a flavorant or sweetener.

In a fourth aspect, the invention provides a method of manufacturing an orally disintegrating tablet comprising the following steps: (a) preparing active pharmaceutical ingredient microparticles; (b) optionally coating the drug microparticles with one or more functional polymers to impart taste-masking and/or controlled release characteristics; (c) preparing a powder mixture comprising polymer coated drug microparticles having a median particle size in the range of 100 to 400 µm from step (b), the micro granules of the first aspect, and other optional pharmaceutically acceptable excipients selected from a flavorant, sweetener, colorant, compression aid, and additional disintegrant; (d) compressing the powder mixture on a rotary tablet press using internal or external lubrication, wherein the orally disintegrating tablet rapidly disintegrates on contact with saliva in the buccal cavity into a smooth, non-gritty, easy-to-swallow suspension containing polymer coated drug microparticles or drug microparticles taste-masked by granulating with a sugar alcohol, super disintegrant and optionally a flavorant or sweetener.

In a fifth aspect, the invention provides a tablet obtained by the method of the third or fourth aspect, wherein the tablet is prepared by a method in which the powder mixture is compressed on a rotary tablet press without the addition of a lubricant to the blend, and the method includes a lubricating device to lubricate material contacting punch surfaces and die wall of the tablet press.

In a sixth aspect, the invention provides a tablet obtained by the method of the third or fourth aspect, wherein the tablet is prepared by a method in which the powder mixture is compressed on a rotary tablet press after mixing with a lubricant selected from the group consisting of magnesium stearate, stearic acid, calcium stearate, zinc stearate, sodium stearyl fumarate, glyceryl behenate.

The orally disintegrating tablets of the invention may be for oral administration without water to the elderly, subjects who find it difficult to swallow conventional tablets/capsules due to dysphagia, children who are unwilling to swallow normal tablets/capsules, 'people on the go', subjects with migraine, severe diabetes or heart conditions, who do not have ready access to water or other drinks.

The active pharmaceutical ingredient(s) which can be used in the present invention is any active ingredient belonging, but not limited, to the class of antipyretic agents, analgesic agents, anti-inflammatory agents, antibiotic agents, antihistamine agents, anti-anxiety agents, anti-migraine agents, antiemetic agents, skeletal muscle-relaxants, smooth muscle relaxants, antiplatelet agents, antidepressants, cardiovascular agents (e.g., antiarrhythmics, antihypertensives, ACE inhibitors, angiotensin II receptor antagonists, β-blockers, calcium channel blockers, and diuretics), antihypnotics / antianxiolytics, opioids, antipsychotic agents, antiAlzheimer drugs, antiallergics, drugs indicated to treat diabetes, gastrointestinal disorders, rheumatoid arthritis, which are prescribed for oral administration. The sugar alcohol may be selected from the group consisting of mannitol, xylitol, maltitol, sorbitol and the like. The saccharide may be selected from the group consisting of lactose, sucrose, fructose, and the like.

It is noted that in this disclosure and particularly in the claims and/or paragraphs, terms such as "comprises", "comprised", "comprising" and the like can mean "includes", "included", "including", and the like; and that terms such as "consisting essentially of' and "consists essentially of" can allow for elements not explicitly recited, but exclude elements that are found in the prior art or that affect a basic or novel characteristic of the invention.

### BRIEF DESCRIPTION OF THE DRAWINGS

The following detailed description, given by way of example, but not intended to limit the invention solely to the specific embodiments described, may best be understood in conjunction with the accompanying drawings, in which:

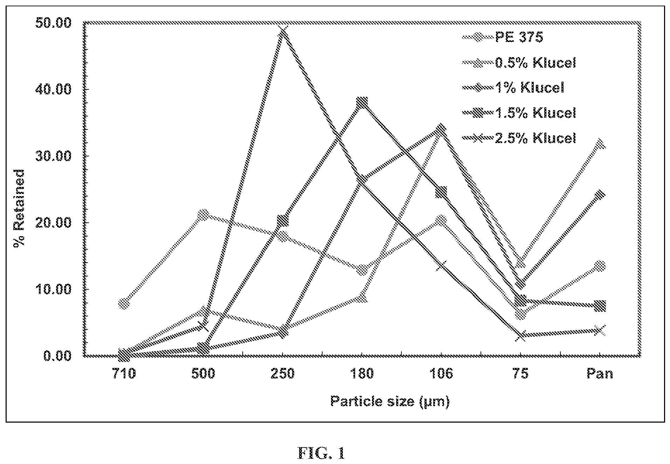

FIG. 1 shows the particle size distributions of rapidly dispersing microgranular compositions, comprising hydroxypropylcellulose as a multi-functional additive at a content of 0.5, 1.0, 1.5, and 2.5% by weight of the microgranule, vs. rapidly dispersing microgranules prepared according to the disclosures in US 20050232988 A1.

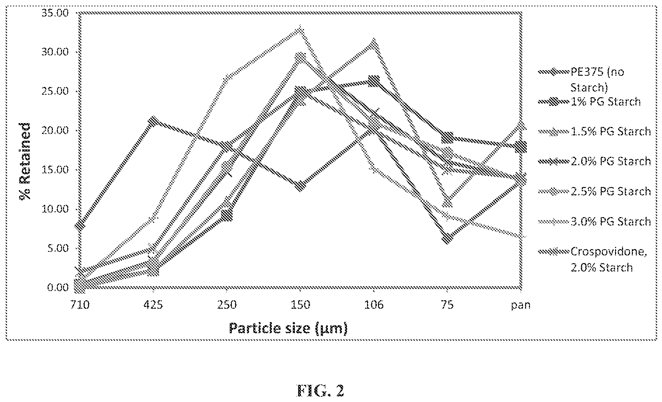

FIG. 2 shows the particle size distributions of rapidly dispersing microgranular compositions, comprising pregelatinized starch as the multi-functional additive at a content of 1.0, 1.5, 2.0, 2.5, and 3.0% by weight of the microgranule, prepared in accordance with certain embodiments of the present invention vs. rapidly dispersing microgranules prepared according to the disclosures in US 20050232988 A1.

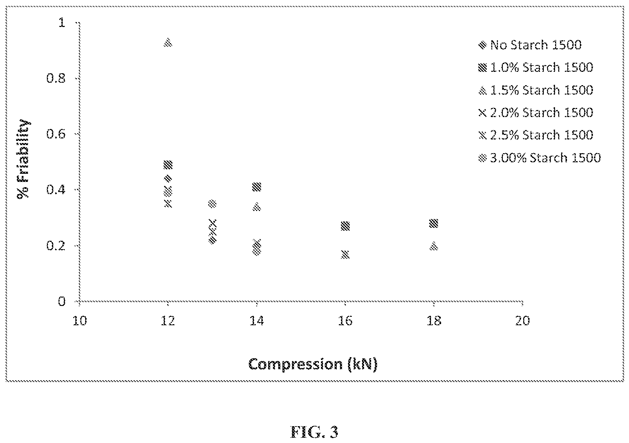

FIG. 3 shows the effect of pregelatinized starch incorporated as the multi-functional additive in the rapidly dispersing microgranular compositions, prepared in accordance with certain embodiments of the present invention, on friability of orally disintegrating tablets prepared according to the disclosure in US 20050232988 A1.

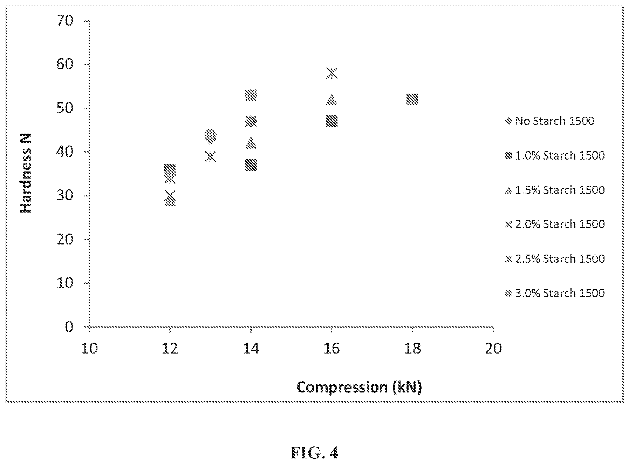

FIG. 4 shows the effect of pregelatinized starch incorporated as the multi-functional additive in the rapidly dispersing microgranular compositions, prepared in accordance with certain embodiments of the present invention, on hardness of orally disintegrating tablets prepared according to the disclosure in US 20050232988 A1.

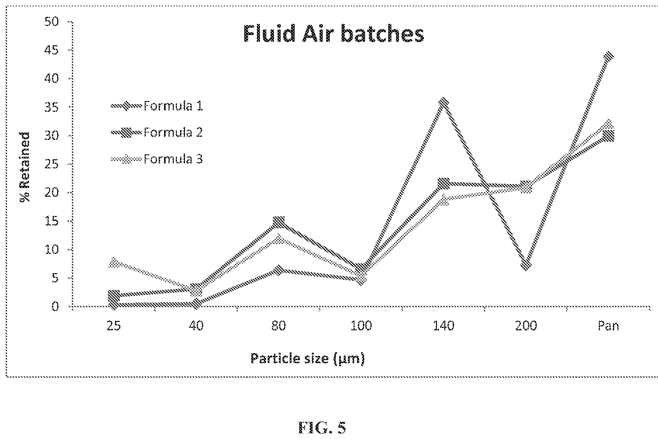

FIG. 5 shows the particle size distributions of rapidly dispersing microgranular compositions, comprising multi-functional additive pregelatinized starch at a content of 2.0% by weight of the microgranule, prepared in commercial Fluid Air FA 300.

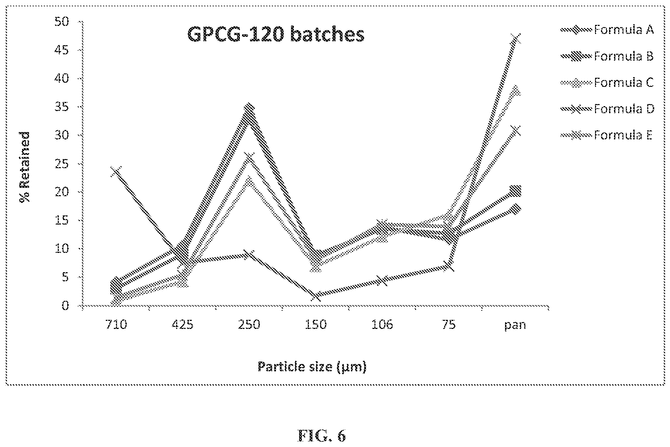

FIG. 6 shows the particle size distributions of rapidly dispersing microgranules, comprising multi-functional additive pregelatinized starch at a content of 2.0% (batch A to D) and 2.5% (batch E) by weight of the microgranule, prepared in commercial Glatt GPCG 120.

### DETAILED DESCRIPTION OF THE INVENTION

The rapidly dispersing microgranules of the invention may include at least one sugar alcohol, saccharide or mixture thereof, at least one super disintegrant selected from the group consisting of crospovidone, croscarmellose sodium, sodium starch glycolate, low-substituted hydroxypropylcellulose and mixtures thereof and pregelatinized starch in a ratio of88-98 (sugar alcohol): 1-10 (disintegrant): 1-3 (pregelatinized starch), for example 88-96 (sugar alcohol): 1-10 (disintegrant): 1-3 pregelatinized starch). The rapidly dispersing microgranules of the invention are suitable for blending with taste-masked and/or controlled release coated microparticles in therapeutically effective amounts at a ratio of from 6:1 to 1:2 for compression into orally disintegrating tablets.

The orally disintegrating tablets produced in accordance with one of the embodiments of the present invention exhibit rapid disintegration in the buccal cavity of a mammal without the need for water or other drinks, in one embodiment, within 60 seconds, i.e., the tablet disintegrates in the saliva in the buccal cavity for the ease of swallowing along with the saliva. In another embodiment, orally disintegrating tablets produced in accordance with the invention disintegrate in the buccal cavity of a mammal within 10 seconds, within 20 seconds, within 30 seconds, within 40 seconds, within 50 seconds or within 60 seconds. Disintegration occurs without the need for water or other drinks.

Thus, the orally disintegrating tablets produced in accordance with one of the embodiments of the present invention meet the disintegration time criteria of not more than 30 seconds when tested by <701> disintegration test method (see Guidance to Industry). Furthermore, the orally disintegrating tablets comprising rapidly dispersing microgranules produced per one of the embodiments of the present invention possess sufficient mechanical strength to resist attrition/chipping during packaging in blisters and bottles, storage and transportation for commercial distribution and end use.

Generally, the term, 'first or primary particle', refers to a particle of the sugar alcohol or saccharide obtained by milling/sieving the raw material. The term, 'secondary particle', refers to a particle of the granulated material, a granulation of the mixture of a sugar alcohol or a saccharide, a disintegrant, and a multifunctional additive, with or without an active ingredient. For example, crystalline mannitol is commercially available with a median particle size of about 60 µm (as Pearlitol® 60 with a bulk density of 0.66 g/mL, a tap density of 0.85 g/mL, and a compressibility of 22.4%), about 35 µm (as Pearlitol® 35 with a bulk density of 0.55 g/mL, a tap density of 0.78 g/mL, and a compressibility of 29.5%), and 15-25 µm (as Pearlitol® 25 with a bulk density of 0.49 g/mL, tap density of 0.74 g/mL, and a compressibility of 33.8%). Low-substituted hydroxypropylcellulose, L-HPC (MS-0.2-0.4) swells in water and is insoluble. L-HPC is used as a super disintegrant in solid medicaments although it can be a binder. Micronized L-HPC is commercially available from Shin Etsu Chemical Co. Limited as L-HPC LH-31 (hydroxypropyl content of 10.0-12.9 %) and L-HPC LH-32 (hydroxypropyl content of 7.0-9.9 %).

The term, 'additive with multi-functionality' or 'multi-functional additive' refers to a pharmaceutically acceptable excipient which has multi-functional activity. For example, starch can act as a binder, a disintegrant, a diluent/filler, a glidant, etc. Starch at a concentration of 5-25% w/w in tablet granulations is widely used as a binder. Pregelatinized starch is starch that has been chemically and/or mechanically processed to render it to be flowable and directly compressible. Hydroxypropylcellulose (HPC with MS=3) is used as a binder, thickening or viscosity increasing, or a coating agent. HPC at concentrations of 2-6% w/w is typically used as a binder in either wet and dry granulations or direct-compression tableting processes.

The term 'free flowing' as it relates to the rapidly dispersing microgranules refers to microgranules being capable of progression or substantially unimpeded movement without forming lumps or aggregates.

The term 'high usable yield' refers to the yield of greater than about 70% by weight, more particularly greater than about 80%, and even more particularly greater than about 90% or as shown in the examples presented herein.

Disclosed herein is a method of producing rapidly dispersing microgranules having an average particle diameter in the range of about 100-300 µm (e.g., by fluid bed granulation), comprising a sugar alcohol (e.g., mannitol with a mean particle size of 60 µm or less, or 50 µm or less, or 40 µm or less, or 30 µm or less) or a saccharide (e.g., lactose monohydrate with a mean particle size of 100 µm or less, or 90 µm or less, or 80 µm or less, or 70 µm or less) in an amount of about 88-98% by weight, a super disintegrant in the amount of about 10-1% by weight, and a pharmaceutically acceptable additive with multi-functionality (e.g., acting as a disintegrant, binder, or diluent) in an amount of about 1-3% by weight of the rapidly dispersing microgranules.

In the methods of manufacturing an orally disintegrating tablet of the invention, the ratio of rapidly dispersing microgranules to that of taste-masked and/or functional polymer coated microparticles of at least one active pharmaceutical ingredient may be from about 6:1 to about 1:2.

The methods of manufacturing an orally disintegrating tablet of the invention may comprise compressing a powder mixture comprising rapidly dispersing microgranules, taste-masked and/or functional polymer coated drug microparticles, and optionally an additional disintegrant, compression aid, flavor, sweetener, and colorant using a rotary tablet press equipped with an external lubrication system to lubricate material contacting punch surfaces and die wall with a lubricant such as magnesium stearate prior to each compression.

Orally disintegrating tablets manufactured by the methods of the invention may disintegrate within 60 seconds on contact with saliva in the buccal cavity of a mammal or within 30 seconds when tested for disintegration time by the United States Pharmacopeia method <701>. For example, the orally disintegrating tablet manufactured by the methods of the invention may disintegrate in the buccal cavity of a mammal within 10 seconds, within 20 seconds, within 30 seconds, within 40 seconds, within 50 seconds or within 60 seconds. Disintegration occurs without the need for water or other drinks.

The methods of manufacturing an orally disintegrating tablet of the present invention may comprise the steps of:
1. granulating with water a mixture comprising a sugar alcohol or a saccharide, each (primary particle), a super disintegrant selected from the group consisting of crospovidone, croscarmellose sodium, sodium starch glycolate, low-substituted hydroxypropylcellulose and mixtures thereof, and pregelatinized starch in a fluid bed granulator, to produce rapidly dispersing microgranules, without requiring milling of moist granulations and extensive milling of dried granules, having a median particle size of about 150 to about 300 µm as measured by using a sonic sifter or a laser particle analyzer;
2. mixing rapidly dispersing microgranules thus produced with taste-masked and/or functional polymer coated drug microparticles, and optional excipients (e.g., a flavor, a sweetener, additional disintegrant, a compression aid, and a lubricant such as sodium stearyl fumarate);
3. compressing the compression mix into orally disintegrating tablets on a rotary tablet press at a comparatively low pressure such that the tablets thus produced not only have adequate mechanical strength to resist attrition/chipping during packaging in PTP (press-through-package) or peel-off paper backed blisters and bottles, storage, transportation, commercial distribution, and end use, but also disintegrate rapidly in the buccal cavity, for example, within 60 seconds, without chewing or the assistance of water or other drinkable fluids. In another embodiment, the tablet produced in accordance with the invention disintegrates in the buccal cavity of a mammal within 10 seconds, within 20 seconds, within 30 seconds, within 40 seconds, within 50 seconds or within 60 seconds. Disintegration occurs without the need for water or other drinks.

Alternatively, a non-lubricated compression mix as described above can be compressed into orally disintegrating tablets on a rotary tablet press by spraying a lubricant onto material contacting surfaces of punches and dies of a tableting machine for ease of tablet compression and ejection. If the pharmaceutical active is not particularly bitter, i.e., if taste-masking with a polymer, a waxy material, or an ion exchange resin is not required to mask the drug taste, drug containing microgranules can be manufactured at industrial scale by granulating a powder mixture comprising a sugar alcohol or a saccharide in the amount of about 60%-96% w/w, a super disintegrant selected from the group consisting of crospovidone, croscarmellose sodium, sodium starch glycolate, low-substituted hydroxypropylcellulose and mixtures thereof in the amount of about 1%-10% w/w, an additive in the amount of about 1%-3% w/w, and the drug in the amount of about 0.1%-30% w/w of the total weight of the drug containing microgranules. These drug-containing microgranules are optionally blended with rapidly dispersing microgranules and other excipients (e.g., a flavor, sweetener, colorant, compression aid, additional disintegrant, and the like) in required amounts and compressed into orally disintegrating tablets with internal or external lubrication.

Further variations include granulating a powder mixture comprising a sugar alcohol or a saccharide, a super disintegrant selected from the group consisting of crospovidone, croscarmellose sodium, sodium starch glycolate, low-substituted hydroxypropylcellulose and mixtures thereof, pregelatinized starch, and an active pharmaceutical ingredient not requiring taste-masking with a polymer, blending the rapidly dispersing microgranules with other pharmaceutical excipients (e.g., a flavor, sweetener, colorant, compression aid, additional disintegrant, and the like) and compressing into orally disintegrating tablets with internal or external lubrication.

Active pharmaceutical ingredients which are suitable for use in the orally disintegrating tablet, include, but are not limited to the following classes of pharmacologically active ingredients approved or approvable for oral administration - drugs for central nervous system (stimulants such as amphetamine, methylphenidate); antidepressants such as citalpram, sertraline, fluoxetine; antiemetics such as ondansetron, palonosetron; cardiovascular agents (antiarrhythmics such as atenolol, pindolol, sotalol; antihypertensive agents such as todrazine, nicardipine, guanfacine; ACE inhibitors such as inalapril, captopril; angiotensin II receptor antagonists such as valsartan, eprosartan; β-blockers such as metoprolol, carvedilol; calcium channel blockers such as amlodipine, nifedipine, verapamil; diuretics such as furosemide, hydrochlorothiazide); antihypnotics / antianxiolytics (e.g., valproate sodium, nitrazepam, phenytoin); sedatives (e.g., clonazepa, temazepam, zolpidem, diphenhydramine); antiepileptics (valproate sodium, nitrazepam, phenytoin, lamotrigine); analgesics / antipyretic agents (e.g., aspirin, acetaminophen, ibuprofen, diclofenac sodium, meloxicam, indomethacin); drugs for rheumatoid arthritis; antimigraine drugs such as sumatriptan, zolmitriptan; opioids such as morphine, fentanyl, oxycodone; drugs for Parkinson's disease (e.g., carbidopa-levodopa, amantadine, hyoscyamine, pramipexole, selegeline, ropinirole); antipsychotic agents (e.g., clozapine, paliperidone, amitriptyline, tropisetron); antiplatelet drugs (e.g., clopidogrel, prasugrel, ticlopidine, dipyridamole, cilostazol); skeletal muscle relaxants (e.g., cyclobenzaprine, clonidine, baclofen, tiznidine, hyoscyamine); anti-Alzheimer drugs (e.g., donezapil, galanthamine); antispasmodic agents (e.g., dicyclomine); proton pump inhibitors / histamine H₂ antagonists (e.g., pantoprazole, lansoprazole, famotidine); drugs to treat gastrointestinal disorders (gastroparesis, Crohn's disease, Ulcerative colitis, inflammatory bowel disease, constipation, diarrhea such as metoclopramide, cisapride, domperidone, aminosalicylates, tegaserod, metronidazole, corticosteroids); antidiabetics (e.g., glimeperide, glipizide, metformin, tolbutamide); antiallergics (e.g., cetirizine, loratidine); antibiotics (e.g., paramomycin, amoxicillin, clarithromycin, azithromycin, cefalexin, minocycline).

A sugar alcohol or a saccharide, each (primary particle) having an average particle diameter of about 60 µm or less, may be used in the preparation of rapidly dispersing microgranules at a pilot/industrial scale, and the amount used in the formulation may vary from about 88 to about 98% by weight of the rapidly dispersing microgranules. If the particle diameter is larger, the sugar alcohol or saccharide may be milled using a jet mill or the like. The sugar alcohol, in one embodiment, is selected from mannitol, xylitol, maltitol, sorbitol, isomalt, erythritol, lactitol, and the like. The saccharide, in one embodiment, is selected from lactose, sucrose, dextrose, fructose, maltose, and the like.

An additional disintegrant suitable for incorporation into the intrabuccally rapidly disintegrating tablet includes a cross-linked polyvinylpyrrolidone (referred to as Polyplasdone or Crospovidone), a cross-linked sodium carboxymethyl cellulose (referred to as Croscarmellose sodium), sodium starch glycolate, low-substituted hydroxypropylcellulose, and the like, which are widely used in drugs and food industry. In one embodiment, the amount of super disintegrant to be used in rapidly dispersing microgranules may be from about 1% to about 10% by weight of the rapidly dispersing microgranules, for example from about 2% to about 8% by weight of the rapidly dispersing microgranules, from about 3% to about 7% by weight of the rapidly dispersing microgranules, or from about 4% to about 6% by weight of the rapidly dispersing microgranules.

In conventional tablets a disintegrant such as crospovidone or starch is used at a level of up to about 25% by weight of the tablet to achieve a disintegration time of not more than 5 min when tested by the United States Pharmacopoeia method <701>. Such tablets are generally not suitable for disintegration in the buccal cavity.

In one embodiment, a lubricant, such as magnesium stearate, calcium stearate, zinc stearate, stearic acid, sodium stearyl fumarate, glyceryl behenate or the like is used for lubricating the granules or externally applied onto material contacting die and punch surfaces of a rotary tablet press used to compress tablets.

The ODT tablets according to the present invention can be obtained by compressing into tablets after granulating a powder mixture comprising a sugar alcohol or a saccharide, pregelatinized starch, and a super disintegrant selected from the group consisting of crospovidone, croscarmellose sodium, sodium starch glycolate, low-substituted hydroxypropylcellulose and mixtures thereof with water, acetone, ethanol, isopropanol, or mixture thereof, blending with drug microparticles coated with one or more functional polymers, hydrophobic waxes, fatty acids, fatty acid esters, and mixtures thereof to impart taste-masking or controlled release characteristics and optional ODT excipients (e.g., a flavor, a sweetener, a disintegrant, a colorant, a compression aid) and compressed into tablets using a rotary tablet press with an internally or externally applied lubricant. Alternatively, the ODT tablets can be manufactured at an industrial scale by granulating a powder mixture comprising a sugar alcohol or a saccharide, each primary particle having an average particle diameter of about 60 µm or less, a pharmaceutically acceptable active ingredient not requiring taste-masking with one or more functional polymers, hydrophobic waxes, fatty acids, fatty acid esters, and mixtures thereof, and a super disintegrant selected from the group consisting of crospovidone, croscarmellose sodium, sodium starch glycolate, low-substituted hydroxypropylcellulose and mixtures thereof using a solution of pregelatinized starch, and compressing the powder mixture comprising rapidly dispersing/dissolving drug-containing microgranules, optional ODT excipients, and additional rapidly dispersing microgranules into an ODT tablet that rapidly disintegrates on contact with saliva in the buccal cavity of a mammal forming a smooth, easy-to-swallow suspension with no aftertaste, or disintegrates within 30 seconds when tested by the USP Disintegration Time test method <701>.

The granulation method is not limited; however a fluid bed granulation method using the solution of the additive dissolved in purified water, ethanol, isopropanol, acetone, or mixtures thereof may be employed. Granulation may be performed by spraying the additive solution onto the powder mixture in a top spray fluid bed granulator such as a Glatt GPCG 5, GPCG 120, or WSG granulator or Fluid Air FA0300, and drying the granulation in the same fluid-bed dryer. The dried granulated material thus produced is sieved by passing through appropriate sieves to collect rapidly dispersing microgranules with a desired particle size distribution by discarding fines and optionally milling/resieving oversized granules. Fluid bed granulation of mannitol and low substituted hydroxypropylcellulose using an aqueous solution of pregelatinized starch in a fluid bed granulator may be undertaken with limited milling steps to effect a total yield of useable rapidly dispersing microgranules with a particle size distribution of not more than about 400 µm of not less than 90% by weight of the total granulations. Furthermore, the number of milling steps that are required to produce useable rapidly dispersing microgranules with a particle size distribution of not more than 400 µm, i.e., milling of moist granulations, milling of partially dried granulations, and sieved oversized granules may be reduced to none or at worst to a single milling of sieved oversized granules, which is typically less than 5 wt.% of the total theoretical batch size. The rapidly dispersing microgranules of the invention may be mixed with coated drug microparticles (e.g., effectively taste-masked and/or controlled release (CR) coated, i.e., drug cores coated with one or more functional polymers to impart desired *in vitro*/*in vivo* drug release properties) and optionally a flavor, sweetener, color, additional disintegrant, and compression aid, and thereafter compressed into a predetermined shape, an orally disintegrating tablet exhibiting rapid disintegration in the buccal cavity, for example, within 60 seconds.

The drug microparticles coated with one or more functional polymers to impart taste-masking and/or controlled release characteristics may have a median particle size in the range of about 100 µm to about 400 µm (for example about 200 µm to about 400 µm, or about 300 µm to about 400 µm, or about 100 µm to about 350 µm). The microparticles may be sized such that no less than 90% of the microparticles are smaller than about 600 µm. This enables an orally disintegrating tablet incorporating such microparticles to result in a smooth, non- gritty mouthfeel when placed in the oral cavity of a human subject. Such taste-masked and/or CR coated drug microparticles can be prepared in accordance with the disclosures in US 6,500,454 B1; US 6,627,223 B1; US 6,663,888 B1; US 20050232988 A1; US 20060078614 A1; US 20060105039 A1; US20060105038 A1; US20070196491 A1; US 20070190145 A1; US 20080069878 A1; US 20090092672 A1; US 20090155360 A1, US 20090169620 A1, US 20090202630 A1; US 20090232885 A1; US 20090258066 A1; US 20100025083 A1; US 20100025067 A1; US Patent Application Ser. No. 12/639,496; US Patent Application Ser. No. 12/688,493; US Patent Application Ser. No. 12/772,770; and/or US Patent Application Ser. No. 12/772,776.

Orally disintegrating tablets of the present invention can be produced by an internal lubrication method wherein the compression mix is further blended with a lubricant prior to compression or an external lubrication method wherein a lubricant is not included in the tablet formulation, but is externally applied onto the material contacting surfaces of punches and dies of a rotary tablet press.

The invention will now be further described by way of the following non-limiting examples.

The following examples provide comparative illustrations of rapidly dispersing (RD) microgranules and orally disintegrating tablets comprising these microgranules, taste-masked and/or CR coated drug microparticles, and optionally other excipients produced in accordance with the present invention in comparison with those produced in accordance with US 20030215500 and/or US 20050232988. Comparative illustrations are also provided of rapidly dispersing drug-containing microgranules comprising a sugar alcohol or a saccharide, each primary particle having an average particle diameter of about 60 µm or less, a pharmaceutically acceptable active ingredient not requiring taste-masking, and a super disintegrant using a solution of an additive with multi-functionality and orally disintegrating tablets comprising these drug-containing microgranules, rapidly dispersing microgranules, and other ODT excipients, in comparison with those produced in accordance with US 20030215500 and/or US 20050232988.

### EXAMPLES

The present invention is further illustrated by reference to the following Examples. However, it should be noted that these Examples, like the embodiments described above, are illustrative and are not to be construed as restricting the scope of the invention in any way.

### Comparative Example 1.A: Solblet Granules in Kogyo FS-200 - Sangyo FLO-120

D-mannitol (38 kg) with a median particle size of about 15 µm and crospovidone (2 kg) are charged into a Kogyo FS-200 granulator following sieving through a 30 mesh screen to deagglomerate, and granulated with purified water at 20% by weight. Granulations from two batches performed under the same conditions are dried in a fluid bed dryer, Sangyo FLO-120 at an inlet temperature of 90°C under a fluidization air volume of 100 cfm to achieve a LOD of less than 1% by weight. The dried granules are sieved to discard oversized granules, if any.

### Comparative Example 1.B: Rapidly Dispersing Microgranules in GMX 600-Glatt 200

For example, D-mannitol (152 kg of Mannitol 25 with a median particle size of about 15 µm from Roquette) and crospovidone (8 kg of Polyplasdone XL-10 from ISP) are charged into a high shear granulator from Vector Corporation, GMX 600 following sieving through a 30 mesh screen to deagglomerate, and granulated with purified water (38 kg). Granulations from two batches performed under the same conditions are vacuum transferred into a fluid bed dryer, Glatt GPCG 200 via a Comil®(wet milled) and dried at an inlet temperature of 90°C under a fluidization air volume to achieve a LOD of less than 1% by weight. The dried granules are sieved by passing through a 20 mesh screen in a Kason siever, oversized granules milled using a Comil, and resieved to collect microgranules with desired particle size distributions.

Table 1 shows the process parameters used for manufacturing rapidly dispersing (RD) microgranules at Kyowa and Eurand in accordance with the disclosures in US 20030215500 A1 and/or US 20050232988 A1.

### Comparative Example 1.C: Rapidly Dispersing Microgranules in Glatt GPCG 5

A Glatt GPCG 5 equipped with a top spray granulator bowl, 1.2 mm nozzle (nozzle tip even with air cap), and a peristaltic pump set to deliver purified water at 100 mL/min, is charged with 6650 g of D-mannitol, each particle having an average particle diameter of not more than 30 µm, and 350 g of crospovidone (Polyplasdone XL-10) and granulated with purified water under following conditions:
Pre-heat conditions: Inlet air flap setting - 50%; Inlet air volume - 300 CFM; Inlet air temperature - 100°C; Final outlet temperature - 70°C. Granulation conditions: Inlet air flap setting - 37%; Inlet air volume - 135-150 CFM; Inlet air temperature - 60°C; Product temperature - 30±0.5°C; Atomization air pressure - 1.0 bar; solution spray rate - 100 mL/min. Drying conditions: Inlet air flap setting - 38%; Inlet air volume - 155 CFM; Inlet air temperature - 100°C; Final outlet temperature - 43°C.

The granulation is dried in the Glatt dryer to a LOD of 0.56% at 85°C as measured using a Compu-Trac moisture analyzer, and the useable yield is very low (< 70% by weight). The sieve analysis is performed using an ATM sonic shifter (10 g sample at intensity setting of 8 and time: 4 min. Bulk and tap density measurements are performed to calculate the compressibility percentage following the USP methodology.

### Comparative Example 1.D: RD Microgranules in Glatt GPCG 120

A Glatt GPCG 120 equipped with a top spray granulator bowl and a top spray gun with 3 heads (three 1.8 mm nozzles) and 3 peristaltic pumps set to deliver purified water at 2000 mL/min to the single gun with three heads, is charged with 152 kg of D-mannitol (each particle having an average particle diameter of not more than 30 µm), and 8 kg of crospovidone (Polyplasdone XL-10) and granulated with purified water under following conditions:
Pre-heat conditions: Inlet air volume - 2500 CFM; Inlet air temperature - 100°C; Final outlet temperature - >60°C. Granulation conditions: Inlet air volume - 2000 CFM; Inlet air temperature - 95°C; Product temperature - 31.5±0.5°C; Atomization air pressure - 1.0 bar; solution spray rate - 2000 mL/min. Drying conditions: Inlet air volume - 1500 CFM; Inlet air temperature - 100°C; Final outlet temperature - > 45°C.

The dried granulation is passed through 20 mesh sieve using a Kason siever. A lot of granulation sticking to the wall of the product bowl that is scraped down, is recorded, and consequently the yield is very low (< 70% by weight). The sieve analysis is performed using an ATM sonic shifter (see Table 1 for the particle size, bulk and tap density results).

### Comparative Example 1.E: RD Microgranules containing Povidone in Glatt GPCG 120

Povidone (K-30; 4.32 kg) is slowly added to purified water in a stainless steel container while constantly stirring to dissolve. A mixture of 150 kg of D-mannitol (median particle size: < 30 µm) and 8.65 kg of Crospovidone (XL-10) is granulated in the Glatt GPCG 120 under following conditions: Granulation conditions: Inlet air volume - 2000 CFM; Inlet air temperature - 95°C; Product temperature - 32±0.5°C; Atomization air pressure - 1.0 bar; solution spray rate - 2000 mL/min. The inner wall of the product bowl is fairly clean of the granulation sticking to the wall and this is reflected in achieving % useable yield of > 95% by weight.

### Example 1.F: Microencapsulation of Acetaminophen

Acetaminophen USP (Granular grade; particle size: 45-80 mesh or 177-350 µm) from Covidien is taste-masked with ethylcellulose (Ethocel Standard Premium 100 from Dow Chemicals) by solvent coacervation in an industrial scale 500-gallon or 1000-gallon system using a computerized recipe for the process. Upon controlled heating to 80°C to allow dissolution of ethylcellulose and controlled cooling to < 30°C, the microcapsule bed is subjected to vacuum filtration and rinsing with cyclohexane to wash off residual polyethylene. The microcapsules were transferred to a fluid bed dryer, subjected to a drying procedure, and dried for a period of 4-6 hrs to reduce the cyclohexane level to not more than 1000 ppm.

### Example 1.G: Orally Disintegrating Tablets

Aspartame (0.67 kg or 0.45% by weight of the tablet), S.D.Grape flavor (0.83 kg or 0.55%) and Crospovidone XL-10 (10.5 kg or 7%) are blended for 10 min in a 2 cu-ft V-blender and passed through a Comil®equipped with a 20 mesh screen at 1400 rpm. The required amounts of acetaminophen microcapsules (41.17 kg or 27.45%), rapidly dispersing (RD) microgranules (96.82 kg or 64.55%), and the pre-blend are blended in the 10 cu-ft blender as per the established procedures. Subsequently, these compression mixes are compressed into 160 mg ODT tablets weighing approximately 620 mg using a Hata tablet press-Matsui Exlub system at 25 rpm and at an average magnesium stearate flow of 2.34 volts (equivalent to a flow rate of 5 g per min). Tablets of each lot are produced for about 30 min at a compression force of 14, 18, 20, 22, 25 and 30 kN. A longer tableting run (up to 4 hrs is also performed at 21-22 kN compression force to evaluate tablet weight and hardness variations with time. The tableting properties are presented in Table 2 at one comparable compression force and in greater detail in Table 3. Placebo ODT tablets comprising pilot scale, semi-industrial scale and industrial scale rapidly dispersing microgranules compressed using the Hata press-Matsui ExLub system exhibit comparable tableting properties.

**Table 1: Granulation / Drying and Processing Conditions**

| **Equipment** | **Kyowa (Ex. 1.B)** | **Eurand(Ex. 1.A)** |
|---|---|---|
| High shear granulator | F. Kogyo FS-200 | Vector GMX 600 |
| - Capacity - Volume (L) | 245 | 600 |
| - Capacity - Load (kg) | 40 | 160 |
| - Water for granulation (%) | 20 | 23.15 |
| - Spray rate (g/kg per min) | 20 | 25 |
| - Spray time (min) | About 10 | About 10 |
| - Atomization Air Pressure (PSI) | 40 | 30 |
| - Impeller speed (RPM) | 120 | 140 |
| - Chopper speed (RPM) | 2000 | 2600 |
| Wet milling (Speed: 1400 RPM) | Not Required | Quadro Comil® (Screen: 0.187") |
| Vacuum Transfer | Not required | Required |
| Fluid bed Dryer | Sangyo FLO-120 | Glatt 200 |
| - Load | 80 kg | 320 kg |
| - Inlet Temperature (°C) | 90-100 | 90-100 |
| - Inlet Air Volume (m³/hr | 3600 | 2500* |
| - Bed Height (cm) | 20-30 | 40-50 |
| - Drying Time (min) | 20 min | < 10* |
| - End point of drying- Outlet Air | 50°C | 45°C |
| Sieving | Sieving (20 mesh) | Kason Siever (20 mesh) |
| Dry milling | Not required | Fitzmill® |
| - Screen Size | NA | 0.62" Round |
| - Speed (RPM) | NA | 1400 |

| | | |
|---|---|---|
| NA - Not applicable * - Partial drying occurs during vacuum transfer; hence requiring lower fluidization air volume and drying time | | |

**Table 2: Comparison of Solblet and RD Microgranules and their Properties**

| **Property** | **Kyowa** | **GMX 600 / Glatt 200 (2x160)¹** | **GMX 600 / Glatt 200 (2x160)²** | **Fluid-bed (No binder)³** | **Fluid-bed (PVP binder)⁴** |
|---|---|---|---|---|---|
| RD Granules lot# | Ex. 1.B) | Ex. 1.A | Ex. 1.A | Ex. 1.D | Ex. 1.E |
| % Water Added | 20 | 23.125 | 23.125 | 77 | 30 |
| Wet-milled? | No | Yes | Yes | No | No |
| Vacuum TT | NA | 85 min | 85 min | NA | NA |
| Drying Time | <10 min | <10 min | <10 min | 10 min | 5 min |
| % LOD at 85°C | 0.48 | 0.05 | 0.05 | 0.75 | 0.80 |
| Useable Yield, % | NA | 70 | 70 | 72 | 93 |
| % Oversized | NA | 21 | 21 | 2.6 | 5 |
| Oversized Used? | NA | No | Yes | No | No |
| Bulk Density (g/mL) | 0.55 | 0.56 | 0.56 | 0.52 | 0.47 |
| Tap Density (g/mL) | 0.71 | 0.67 | 0.67 | 0.69 | 0.58 |
| Compressibility (%) | 22.8 | 17.3 | 17.3 | 24.1 | 18.2 |

| Particle Size Distribution (%) | | | | | |
|---|---|---|---|---|---|
| >300 µm (50 mesh) | 13.5 | 28.6 | 28.6 | 12.4 | 14.7 |
| <300 µm ->106 µm (140 mesh) | 44 | 43.8 | 43.8 | 36.1 | 49 |
| <106 µm | 41 | 27.7 | 27.7 | 51.5 | 36.4 |

| Tableting Properties | | | | | |
|---|---|---|---|---|---|
| Tableting lot# | Formula 1 | Formula 2 | Formula 3 | Formula 4 | Formula 5 |
| RD Granules lot# | Ex. 1.A | Ex.1.B | Ex. 1.B | Ex. 1.D | Ex. 1.E |
| Compress Force(kN) | 21 | 21 | 21 | 21 | 22 |
| Weight (%RSD) | 622 (0.6) | 620 (0.55) | 623 (0.88) | 619 (0.35) | 614 (0.75) |
| Hardness (N) | 70 (7.9) | 77 (5.2) | 75 (7.0) | 67 (7.1) | 109 (6.0) |
| Friability (%) | 0.58 | 0.46 | 0.42 | 0.53 | 0.70 |
| Disintegrat Time (sec) | 32 | 38 | 35 | 25 | 53-160 |

| | | | | | |
|---|---|---|---|---|---|
| *¹ RD Granules lot (Glatt drying process with 2x160 kg GMX 600 batches) not containing milled oversized material* *² Same granulation lot containing the oversized material after milling and sieving* *³ Fluid-bed processed RD granules lot not containing a binder (total granulation*/*drying time* ∼ *60 min)* *⁴ Fluid-bed processed granulation lot containing a binder, Povidone at 2.7% by weight (total granulation*/*drying time* ∼ *60 min)* | | | | | |

**Table 3: Tableting properties of ODT Formulations**

| **Tablet #** | **RD Granules #** | **Tableting & Properties** | | | |
|---|---|---|---|---|---|
| | | **Compression Force (KN)** | **Weight, mg (%RSD)** | **Hardness (N) (%RSD)** | **Friability (%)** |
| Formula 1 | Ex. 1.A | 14.5 | 615 (0.24) | 40 (7.1) | 1.39 |
| | | 18.2 | 620 (0.47) | 58 (7.4) | 0.87 |
| | | 19.5 | 626 (0.58) | 69 (7.5) | 0.58 |
| | | 22.3 | 623 (0.25) | 85 (5.0) | 0.58 |
| | | 25.3 | 623 (0.17) | 94 (5.6) | 0.42 |
| | | 29.7 | 625 (0.24) | 113 (5.9) | 0.32 |
| Formula 2 | Ex. 1.B | 14.1 | 613 (0.44) | 38 (5.4) | 1.30 |
| | | 18.1 | 618 (0.26) | 55 (5.0) | 0.69 |
| | | 21.2 | 621 (0.55) | 75 (5.2) | 0.51 |
| | | 22.0 | 622 (0.74) | 75 (7.4) | 0.47 |
| | | 25.2 | 618 (0.68) | 94 (4.6) | 0.29 |
| | | 30.4 | 619 (0.41) | 121 (6.0) | 0.20 |
| Formula 3 | Ex. 1.B | 14.2 | 617 (0.31) | 37 (9.5) | 1.40 |
| | | 17.9 | 616 (0.52) | 54 (7.4) | 0.88 |
| | | 21.1 | 626 (0.88) | 72 (5.8) | 0.48 |
| | | 22.0 | 620 (0.47) | 78 (8.7) | 0.47 |
| | | 25.4 | 618 (0.48) | 93 (4.9) | 0.36 |
| | | 30.3 | 625 (0.52) | 121 (6.4) | 0.19 |
| Formula 4 | Ex. 1.D(FB Granules) | 13.9 | 616 (0.21) | 34 (5.2) | 1.90 |
| | | 18.3 | 616 (0.45) | 51 (3.7) | 0.80 |
| | | 20.9 | 619 (0.29) | 65 (5.1) | 0.53 |
| | | 22.3 | 618 (0.27) | 69 (6.1) | 0.58 |
| | | 25.2 | 620 (0.37) | 86 (7.9) | 0.41 |
| | | 30.2 | 620 (0.17) | 106 (5.4) | 0.32 |
| Formula 5 | Ex. 1.E (Granules with a binder) | 15.6 | 609 (0.53) | 62 (4.9) | 0.71 |
| | | 17.8 | 615 (0.49) | 71 (6.7) | 0.83 |
| | | 22.0 | 618 (0.41) | 109 (6.0) | 0.63 |

### Physical and Tableting Properties of RD Microgranules

- The particle size distributions vary significantly between GMX-Glatt granulations, Fluid bed (FB) granulations with and without a binder, and also between semi-industrial scale Kyowa Solblet and industrial scale GMX-Glatt 200 granulations.
- Bulk density and shapes of the GMX-Glatt granulations are similar to that of Kyowa Solblet granulation.
- In spite of the differences in particle shape, particle size distribution and/or compressibility, no flow related issues are encountered during the tableting runs of Acetaminophen ODTs (Formula 1 to 5) requiring adjustments of the compression parameters.

- The fill weight variations are held tight with an RSD of less than 1%.
- The variations in tablet hardness are held tight with an RSD of less than 10%.
- The compression mixes containing any of the granulations exhibited similar tableting properties, i.e., tablet weight variation, thickness and hardness), irrespective of whether it is a Kyowa Solblet or GMX-Glatt granulation, or whether the milled, oversized material is blended with the sieved granules or not.
- In the compression force range of 18 to 30 kN, the tablet friability values are not statistically different (see Table 3).
- The fluid-bed granulation (no binder) exhibit marginally lower tablet hardness values than the GMX-Glatt lots. However, the greatest drawback of the fluid bed microgranules is the extensive material loss due to sticking of the mannitol powder to the product bowl surface, thereby resulting in an extremely low total useable yield.
- The tablets of the long tableting runs display similar disintegration times (range: 25-38 sec) - 32 sec for Formula 1 (Kyowa Solblet granules), 38 sec for Formula 2, 32 sec for Formula 3, and 25 sec for Formula 4 (Fluid-bed).
- The fluid-bed granulation with the binder exhibit higher tablet hardness values than the other non-binder containing lots at comparable compression forces (see Tables 2-3). Furthermore, the greatest drawback of the fluid bed microgranules containing a binder is that the ODT tablets exhibit significantly, rather unacceptably longer disintegration times. Thus, from the regulatory and/or financial considerations, both fluid bed processes are considered unsuitable for the manufacture of rapidly dispersing microgranules at industrial scale, thereby creating an unmet need.

### Comparative Example 2.A - Lamotrigine ODTs, 25, 50, 100, and 200 mg

US 20090092672 A1 teaches the method of manufacturing orally disintegrating tablets comprising rapidly dispersing microgranules (Ex. 1.F) and taste-masked lamotrigine crystals at industrial scale. A 500-gallon solvent coacervation system (326 gallons or 1234 L of cyclohexane) is charged with lamotrigine microcrystals (78.3 kg), Ethylcellulose (Ethocel 100 cps; 13.8 kg), Epolene (9.2 kg) and the lamotrigine is taste-masked by solvent coacervation while agitating at 80±5 rpm. A computer controlled "heat to 80°C-and hold" cycle is used to achieve a temperature of 80°C to dissolve the ethylcellulose in the coacervation system. Thereafter the system is subjected to a cooling cycle to < 30°C in not less than 45 min while constantly stirring to avoid the formation of agglomerates. As the temperature fell below about 65°C, the ethylcellulose which is no longer soluble in cyclohexane started precipitating out (assisted by the phase inducer, polyethylene), thereby encapsulating the lamotrigine microcrystals with a smooth coating to provide taste-masking. The microcapsules are vacuum-filtered, washed with cyclohexane, and dried in a fluid bed dryer using a 3-step temperature (e.g., 25°C, 35°C, 99°C) for 4 to 6 hrs to achieve a residual cyclohexane level of less than 1000 ppm. The microcapsules are sieved through a US 35 mesh sieve to discard agglomerates, if any.

Sucralose (0.40% w/w), and crospovidone XL-10 (5.0% w/w) are pre-blended by passing the mixture through a Comil® to achieve homogeneity. Similarly, cherry flavor (1.0% w/w) is pre-blended with a small amount of the rapidly dispersing microgranules (64.19% w/w), and the two pre-blended mixtures are blended until homogeneous. The taste-masked microparticles (29.41% w/w) and the remaining rapidly dispersing microgranules are blended together and further blended with the above pre-blends with a batch size of 160 kg to 550 kg are manufactured. During the industrial scale tablet manufacturing of ODT tablets 25 mg (7 mm x 100 mg), 50 mg (9 mm x 200 mg), 100 mg (11 mm x 400 mg), and 200 mg (14 mm x 800 mg) using a Hata press-Matsui ExLub system, no material flow or compression-related tableting issues are observed.

### Comparative Example 2.B - Acetaminophen ODTs, 250 and 500 mg

US Patent Application Ser. No. 12/772,770 or US Patent Application Ser. No. 12/772,776 teaches the method of manufacturing orally disintegrating tablets comprising rapidly dispersing microgranules and taste-masked acetaminophen crystals at industrial scale. A 500-gallon coacervation system (single tank) is charged with 326 gallons of cyclohexane, 180 kg of acetaminophen (Semi-fine grade A137 from Covidien), 20-24.5 kg of ethylcellulose (Ethocel Standard Premium 100 from Dow Chemicals Co.), and 4.0-4.9 kg of polyethylene while stirring at 60±5 rpm. The system is subjected to a computer controlled "heat and cool" cycle with a holding time at 80°C of about 5 min to microencapsulate the drug-layered beads at an ethylcellulose coating of as is disclosed in Comparative Example 2.A, above.

During the feasibility development of ODT tablets, 250 and 500 mg weighing 700 and 1400 mg, respectively, using a rotary tablet press equipped with an external lubrication system and 13 mm and 17 mm round, flat faced, radius-edge tooling at different compression forces and turret speeds, it is surprisingly found that the compression blend comprising taste-masked acetaminophen at higher than 30% w/w, the industrial scale rapidly dispersing microgranules and crospovidone at 5 wt.%, should include about 10% by weight of microcrystalline cellulose (Avicel PH101) for trouble-free industrial scale tablet manufacturing of such ODT formulations. Accordingly, Aspartame (2,56 kg), Artificial Strawberry flavor (2.56 kg), microcrystalline cellulose (16 kg of Avicel PH101) and crospovidone XL-10 (8 kg) are pre-blended in a 2 cu-ft V blender for 10 min to achieve homogeneity after individually passing through a Comil® to deagglomerate. The taste-masked microparticles (63.5 kg), pre-blend, and rapidly dispersing microgranules (67.4 kg from Ex. 1.F) are blended in a 10 cu-ft V-blender for 15 min to manufacture a compression blend with a batch size of 160 kg. The ODT tablets, 250 and 500 mg having sufficiently high tensile strength and low friability to withstand attrition during packaging in HDPE bottles, storage and overseas shipping for marketing in Europe are compressed. These tablets not only disintegrate within 30 seconds when tested by USP DT method <701> but also released not less than 85% in 15 min when tested using the USP apparatus 2 (paddles@ 75 rpm in pH 5.8 buffer.

### Comparative Example 2.C - Ranitidine HCl ODTs, 75 and 150 mg

US 20090202630 teaches the method of manufacturing orally disintegrating tablets comprising rapidly dispersing microgranules and taste-masked ranitidine HCl crystals at industrial scale Ranitidine HCl microcrystals (Form II) are charged into the 5-gallon system along with Ethocel 100 cps and Epolene and microencapsulated for a coating of 15% by weight while stirring at the speed of 150 rpm in accordance with the disclosures above. The taste-masked ranitidine microparticles are applied an optional flavor coating to minimize the impact of accidental biting into taste-masked drug particles by the pediatric population by spraying a homogenized suspension containing a cherry or vanilla mint flavor (62%) and sucralose (17%), and triethylcitrate (21%), a plasticizer while maintaining a target product temperature of about 41°C. Following the flavor coating, fluid bed coating is continued by spraying Ethocel 10 cps/Eudragit E100/ triethylcitrate solution at a product temperature of 45°C, and the coated drug particles are dried in the unit for 10 min to drive of residual solvents.

Sucralose (0.35 wt.%), cherry flavor (1.3%), Red/Blue colorant (0.5%) microcrystalline cellulose (10% of Avicel PH101) and crospovidone XL-10 (5%) are pre-blended in a V blender for 10 min to achieve homogeneity after individually passing through a Comil® to deagglomerate. The taste-masked microparticles (~ 28%), the pre-blend, and the rapidly dispersing microgranules (-55%) are blended in a V-blender for 15 min and compressed to manufacture ODT tablets, 150 and 75 mg (as free ranitidine) having sufficiently high tensile strength and low friability to withstand attrition during packaging in HDPE bottles or blisters, storage, transportation, commercial distribution, and end use, with no material flow or compression-related tableting issues.

### Comparative Example 2.D - Diphenhydramine HCl ODTs, 25 mg

US 20090155360 teaches the method of manufacturing orally disintegrating tablets comprising rapidly dispersing microgranules and taste-masked diphenhydramine HCl crystals at industrial scale. Hydroxypropylcellulose (8.42 kg of Klucel-LF) is slowly added to an acetone/purified water (86.4 kg/9.6 kg) mixture in a stainless steel tank equipped with a heating jacket at 65°C while agitating at 750±25 rpm until dissolved. Diphenhydramine HCl (76.5 kg) is slowly added to an acetone/purified water (300 kg/93 kg) mixture in another stainless steel tank while agitating at 850±25 rpm until dissolved. The hydroxypropylcellulose solution is slowly added to the drug solution while stirring to homogenize. Sugar spheres (60-80 mesh or 170-250 µm; 215 kg) are charged into a preheated Glatt GPCG 120 fluid-bed coater equipped with a 32" bottom spray Wurster insert. When the beads are properly fluidized, i.e., properly suspended in air, the drug is layered onto sugar spheres by spraying the solution at a spray rate of about 1500 g/min (range: 300-2000 g/min) under processing conditions per computer controlled recipe - Process air volume: 1500 CFM; Atomization air pressure: 2.5 bar with nozzle port size of 1.3 mm (HS collar); Product temperature: 49-51°C - to ensure that the drug layering is continued to completion without spray drying or forming agglomerates. Following the completion of the drug layering, a seal coating of hydroxypropylcellulose is applied at a spray rate of 300 g/min for a 2% weight gain, and the drug-layered beads are dried in the same unit to drive off residual solvents and sieved through #32 and #80 mesh screens to discard oversized particles and fines.

A 200-gallon coacervation system is charged with 150 gallons of cyclohexane, 65.1 kg of drug-layered beads, 6.5 kg of ethylcellulose (Ethocel Standard Premium 100 from Dow Chemicals Co.), and 8.9 kg of polyethylene while stirring at 60±5 rpm. A computer controlled "heat to 80°C-and hold" cycle is used to achieve a temperature of 80°C to dissolve the ethylcellulose in the coacervation system. Thereafter the system is subjected to a cooling cycle to < 30°C in not less than 45 min while stirring to avoid the formation of agglomerates. As the temperature falls below about 65°C, the ethylcellulose which is no longer soluble in cyclohexane starts precipitating out (assisted by the phase inducer, polyethylene), thereby encapsulating the acetaminophen crystals with a smooth coating at 6% by weight to provide taste-masking. The microcapsules are vacuum-filtered, washed with cyclohexane, and dried in a fluid bed dryer using a 3-step temperature (e.g., 25°C, 35°C, 99°C) for 4 to 6 hrs to achieve a residual cyclohexane level of less than 1000 ppm. The microcapsules are sieved through a US 35 mesh sieve to discard agglomerates, if any.

During the feasibility development of ODT tablets, 25 mg weighing 650 mg using a rotary tablet press equipped with an external lubrication system and 11 mm round, flat faced, radius-edge tooling at different compression forces and turret speeds, it is surprisingly found that the use of microcrystalline cellulose (Avicel PH101) at least at about 15-20% by weight would be greatly beneficial in achieving higher tensile strength without affecting the disintegration time or organoleptic properties of the ODT tables.

### Comparative Example 2.E - Acetaminophen/Hydrocodone Bitartrate ODTs, 500-mg/5-mg & 300-mg/10-mg

US Patent Application Ser. No. 12/772,770 or US Patent Application Ser. No. 12/772,776 teaches the method of manufacturing orally disintegrating tablets comprising rapidly dispersing microgranules and taste-masked hydrocodone bitartrate layered onto acetaminophen microcapsules which are acetaminophen crystals taste-masked by solvent coacervation at industrial scale. A 200-gallon solvent coacervation system (cyclohexane: 142 kg) is charged with acetaminophen (Semi-fine grade A137; 75.5 kg), Ethylcellulose (EC-100; 4.8 kg), Epolene (2.1 kg) and the acetaminophen is taste-masked by solvent coacervation in a 200-gallon system while agitating at 80±5 RPM. Using the computer controlled recipe, acetaminophen microcrystals are coated at 6% by weight as disclosed in Comparative Example 2.D, above. Using a similar procedure, acetaminophen microcrystals (94.1 kg) are also taste-masked at 10% by weight with Ethocel 100 cps (10.5 kg) and Epolene (2.1 kg) as the phase inducer.

Hydrocodone bitartrate (3.6 kg) is layered onto acetaminophen microcapsules (at 6% coating; 56 kg) from above by spraying a drug-layering formulation (10% solids) comprising hydroxypropylcellulose (0.4 kg) under optimized processing conditions in a Fluid Air FA-300 fluid bed coater equipped with an 18" bottom spray Wurster insert. Following the drug layering, the microparticles are sealant coated with hydroxypropylcellulose (3.2 kg) and sodium stearyl fumarate (0.5 kg) in the same unit, followed by a taste-masking sucralose (3.3 kg) solution coating and drying for 5 min to reduce residual moisture and sieved through 30 and 80 mesh sieves to discard over sized particles and fines.

During the feasibility development of Acetaminophen/Hydrocodone Bitartrate ODT tablets, 500-mg/5-mg and 300-mg/10-mg weighing 1400 and 1100 mg, respectively, using a rotary tablet press equipped with an external lubrication system and 15 mm and 17 mm round, flat face radius edge tooling with logos at different compression forces and turret speeds, it is surprisingly found that the compression blend comprising taste-masked acetaminophen microcrystals (10% w/w), taste-masked hydrocodone/acetaminophen microparticles, the industrial scale rapidly dispersing microgranules and crospovidone at 5 wt.%, should include a compression aid, microcrystalline cellulose (Avicel PH101) and a material flow enhancer, spray-dried mannitol (Parteck M 300), both at least at 10% by weight for trouble-free industrial scale tablet manufacturing of such acetaminophen/hydrocodone bitartrate ODT formulations. Accordingly, sucralose (2.25 kg), artificial cherry flavor (2.55 kg), microcrystalline cellulose (15 kg of Avicel PH101), spray-dried mannitol (15 kg of Parteck M 300) and croscarmellose sodium (1.5 kg of Ac-Di-Sol) are pre-blended in a 2 cu-ft V-blender for 5 min to achieve homogeneity followed by passing the pre-blend through a Comil® screen/spacer running at 1446 rpm to deagglomerate. The taste-masked hydrocodone/acetaminophen microparticles (9.98 kg for 500-mg/5-mg ODT or 25.39 kg for 300-mg/10-mg ODT), taste-masked acetaminophen microcrystals (50.81 kg for 500-mg/5-mg ODT or 23.29 kg for 300-mg/10-mg ODT), the pre-blend, and the rapidly dispersing microgranules (51.41 kg for 500-mg/5-mg ODT or 63.52 kg for 300-mg/10-mg ODT)) are blended in a 10 cu-ft V-blender for 20 min followed by blending with pre-screened sodium stearyl fumarate (1.5 kg) for 5 min. The ODT tablets, 500-mg/5-mg and 300-mg/10-mg having sufficiently high tensile strength and low friability to withstand attrition during packaging in HDPE bottles or blisters, storage, transportation, commercial distribution, and end use, are manufactured by compressing the compression blend (each 150 kg). These tablets not only disintegrate within 30 seconds when tested by USP DT method <701> but also release both actives not less than 80% (Q) in 30 min when tested using the USP apparatus 2 (paddles@ 50 rpm in pH 5.8 buffer).

### Comparative Example 2.F - Temazepam ODTs, 7.5, 15, 22.5, and 30 mg

US 20090169620 teaches the method of manufacturing orally disintegrating tablets (ODT) comprising rapidly dispersing microgranules and temazepam microgranules at industrial scale. Temazepam microgranules are prepared by granulating in a Glatt GPCG 5 fluid bed granulator temazepam microcrystals, mannitol, and crospovidone using purified water as the granulating fluid (batch size of 6 kg). Various ODT compositions that are prepared by first pre-blending sucralose, cherry or peppermint flavor, crospovidone XL-10, and microcrystalline cellulose, then blending this mixture with the rapidly dispersing microgranules and the temazepam microgranules, are evaluated to determine the "robustness" of the formulations.

Mannitol 25 with a median particle size of about 15 µm (122.4 kg) and crospovidone XL-10 (8.0 kg) are milled by passing the mixture through a Comil® mill. The mannitol, crospovidone, microcrystalline cellulose (Avicel PH 101; 8 kg), and temazepam microcrystals (Covidien, 19.2 kg) are granulated in a Fluid Air FA 300 fluid bed granulator by spraying hydroxypropylcellulose (Klucel LF, 2.4 kg) solution in 3 loops with different air flow volumes and filter bag shaking times to minimize the quantity of fines in the resulting granulation. After spraying, the wet granules are dried for a LOD of < 2.0%. The dried granules are passed through a 20 mesh market grade screen using a Kason 30 sifter to discard oversized aggregates, if any. The process produces temazepam microgranules with very uniform particle size distributions and very high yields (e.g., 96% to 99%).

These results show reduced levels of sticking and fines, and no scoring is observed on any of the tablets blended with microcrystalline cellulose.

### Comparative Example 1A: RD Microgranules comprising Crospovidone and Klucel:

Hydroxypropylcellulose, Klucel LF (90 g) is slowly added to purified water in a stainless steel container while continuously stirring to dissolve. The Glatt GPCG 5 is set up with a top spray product bowl, spray gun, and peristaltic pump. D-mannitol with a median particle size of < 20 µm (5610 g) and Crospovidone (300 g) are granulated by spraying the Klucel solution under following conditions: Granulation conditions: Inlet air volume - 70 scfm; Inlet air temperature - 95°C; Product temperature - 41±1°C; Atomization air pressure - 1.5 bar; solution spray rate - 80 mL/min. The inner wall of the product bowl is fairly clean with the granulation sticking to the wall and this is reflected in achieving % useable yield of > 95 wt.%. The dried material (Formula A) with an LOD of 0.3% is passed through a # 20 mesh screen to achieve > 95% total yield. Granulations are also performed at different Klucel contents (e.g., 2.5%, 0.5%, and 1.0% by weight of the granulation; see Table 4 for actual compositions). The particle size distributions that are obtained in each of the four granulations are measured using a Sonic shifter while the bulk and tap density values are also determined. From these values, percent compressibility values are calculated. Table 4 and FIG. 1 present the particle size distribution data for the 4 RD microgranule batches comprising mannitol/crospovidone/Klucel LF (at 0.5, 1.0, 1.5, or 2.5%) in comparison to that of the PE375 batch (mannitol/crospovidone; no multi-functional additive) manufactured at industrial scale in accordance with US Patent 20050232988.

### Comparative Example 1B: Orally Disintegrating Tablets

Crospovidone, microcrystalline cellulose (Avicel PH101), sucralose, and strawberry flavor are mixed in a polyethylene bag and passed through 40 mesh screen. The screened material is blended with the required amounts of acetaminophen microcapsules (lot# 1198-JMC-106), rapidly dispersing granules comprising hydroxypropylcellulose (Klucel LF) as the binder (Formula K (1.0%), Formula K (1.5%), or Formula K (2.5%)) and/or rapidly dispersing granules without a binder (from Example 1.F) in a 0.25 cu-ft V-blender for 10 min (see Table 5 for 250 mg Acetaminophen ODT compositions and tableting properties).

### Example 2: RD Microgranules (Crospovidone and Starch 1500):

Pregelatinized starch (Starch 1500® from Colorcon at 2% by weight or 120 g) is slowly added to purified water in a stainless steel container while continuously stirring to dissolve. The Glatt GPCG 5 is set up with a top spray product bowl, spray gun, and peristaltic pump to deliver at 85 mL/min. D-mannitol with a median particle size of < 20 µm (5580 g) and Crospovidone (300 g) are granulated by spraying the starch solution under following conditions: Granulation conditions: Inlet air volume - 70 scfm; Inlet air temperature - 95°C; Product temperature - 37±1°C; Atomization air pressure - 1.0 bar; solution spray rate - 80-90 mL/min. The inner wall of the product bowl is fairly clean with the granulation sticking to the wall. The dried material (CS- 2%) is passed through a # 20 mesh screen to achieve a useable yield of 91.3% and 3.8% oversized granules.

**Table 4: RD Microgranules - Compositions and Granule Properties**

| **Ingredients** | | **Composition (g/batch)** | | | |
|---|---|---|---|---|---|
| | | Formula K | Formula K | Formula K | Formula K |
| | | (0.5) | (1.0%) | (1.5%) | (2.5%) |
| D-Mannitol | | 5670 | 5640 | 5610 | 5550 |
| Crospovidone | | 300 | 300 | 300 | 300 |
| Klucel LF | | 30 | 60 | 90 | 150 |
| Purified Water | | 2100 | 2900 | 2900 | 5000 |
| | | | | | |
| LOD (%) | | 0.34 | ND | 0.42 | 0.39 |
| Oversize | | NA | NA | NA | 4.5 g |
| Useable Yield (%) | | 95 | 99 | 97 | 91.6 |
| Sonic sifter Screen Size | | | | | |

| (mesh) | (microns) | **Retained on Sieve (%)** | | | |
|---|---|---|---|---|---|
| 25 | 710 | 0.41 | 0.00 | 0.00 | 0.41 |
| 40 | 420 | 6.85 | 1.01 | 1.22 | 4.45 |
| 60 | 250 | 3.94 | 3.46 | 20.33 | 48.79 |
| 80 | 180 | 8.92 | 26.42 | 38.00 | 25.91 |
| 140 | 150 | 33.82 | 34.15 | 24.60 | 13.56 |
| 200 | 75 | 14.11 | 10.77 | 8.33 | 3.04 |
| Pan | < 75 | 31.95 | 24.19 | 7.52 | 3.84 |
| | | | | | |
| Bulk Density (g/cc) | | 0.44 | 0.39 | 0.42 | 0.44 |
| Tap Density (g/cc) | | 0.54 | 0.48 | 0.52 | 0.53 |
| Compressibility (%) | | 20.00 | 18.89 | 18.89 | 16.67 |

**Table 5: Compositions and Tableting Properties of Acetaminophen ODTs**

| **Ingredient** | **Composition (mg/tablet)** | | | |
|---|---|---|---|---|
| Tablet Lot# | Formula 6 | Formula 7 | Formula 8 | Formula 9 |
| Acetaminophen Microcaps (6% EC-100 Coating) | 274.7 | 274.7 | 274.7 | 274.7 |
| RD Microgranules | 313.3 | 156.6 | 156.6 | 156.6 |
| RD Microgranules (Formula K (1%)) | 0.0 | 156.6 | 156.6 | 156.6 |
| Avicel PH101 | 70.0 | 70.0 | 70.0 | 70.0 |
| Crospovidone | 35.0 | 35.0 | 35.0 | 35.0 |
| Sucralose | 2.8 | 2.8 | 2.8 | 2.8 |
| Strawberry Flavor | 4.2 | 4.2 | 4.2 | 4.2 |
| Mag. stearate | Trace | Trace | Trace | Trace |
| Tablet Weight (mg) | 700.0 | 700.0 | 700.0 | 700.0 |
| | | | | |
| Compression Force (kN) | | 11 | 11 | 11 |
| Weight (%RSD) | | 2.9 | 2.7 | 3.0 |
| Hardness (N) | | 34 | 36 | 38 |
| Friability (%) | | 0.3 | 0.2 | 0.2 |
| Disintegration Time (sec) | | 54 | 40 | 50 |

### Comparative Example 3: RD Microgranules containing L-HPC

D-mannitol with a median particle size of < 20 µm (4750 g) and low-substituted hydroxypropylcellulose (250 g of L-HPC from Shin Etsu Chemical Co., Limited) are granulated in the preheated (90°C) Glatt 5 by spraying purified water under following conditions:
Granulation conditions: Inlet air volume - 75 scfm; Inlet air temperature - 90°C; Product temperature - 39±2°C; Atomization air pressure - 1.0 bar; solution spray rate - 85-95 mL/min.

### Comparative Example 3.A: RD Microgranules containing L-HPC and Klucel LF

Klucel LF (90 g) is slowly added to purified water in a stainless steel container while continuously stirring to dissolve. The Glatt GPCG 5 is set up with a top spray product bowl, spray gun, and peristaltic pump to deliver at 85 mL/min. D-mannitol with a median particle size of < 20 µm (5610 g) and low-substituted hydroxypropylcellulose (300 g LS-HPC) are granulated in the preheated (90°C) Glatt 5 by spraying Klucel solution under following conditions: Granulation conditions: Inlet air volume - 72-75 scfm; Inlet air temperature - 85°C; Product temperature - 39±1°C; Atomization air pressure - 1.0 bar; solution spray rate - 85-94 mL/min The inner wall of the product bowl is clean with no granulation sticking to the wall. The dried material (Formula LK (1.5%)) is passed through a # 20 mesh screen to achieve 96.3% useable yield.

### Example 3.B: RD Microgranules containing L-HPC and Starch 1500:

Pregelatinized starch from National Starch Corp. (120 g) is slowly added to warm water at 50°C in a stainless steel container while continuously stirring to dissolve. The Glatt GPCG 5 is set up with a top spray product bowl, spray gun, and peristaltic pump to deliver at 80 mL/min. D-mannitol with a median particle size of < 20 µm (5580 g) and low-substituted hydroxypropylcellulose (300 g) are granulated in the preheated (90°C) Glatt 5 by spraying the starch solution under following conditions: Granulation conditions: Inlet air volume - 70 scfm; Inlet air temperature - 90°C; Product temperature - 39±2°C; Atomization air pressure - 1.0 bar; solution spray rate - 80-100 mL/min. The inner wall of the product bowl is clean with no granulation sticking to the wall. The dried material (Formula LS (2%)) is passed through a # 20 mesh screen to achieve > 96.4% useable yield and 39 g oversized granules. Granulations are also performed at two starch contents (1.0 and 3.0% by weight of the granulation).

### Example 4.A: RD Microgranules containing L-HPC/Starch 1500

Pregelatinized starch with the trademark, Starch™ 1500 from Colorcon, Inc. as the granulation additive (120 g equivalent to 2% based on the weight of the micro granule) is slowly added to purified water in a stainless steel container while continuously stirring to dissolve. The Glatt GPCG 5 is set up with a top spray product bowl, spray gun, and peristaltic pump to deliver at 85 mL/min. D-mannitol with a median particle size of < 20 µm (5580 g) and low-substituted hydroxypropylcellulose (300 g) are granulated by spraying the starch solution under following conditions: Granulation conditions: Inlet air volume - 75 scfm; Inlet air temperature - 95°C; Product temperature - 37±1°C; Atomization air pressure - 1.0 bar; solution spray rate - 85-100 mL/min. The inner wall of the product bowl is fairly clean with the granulation sticking to the wall. The dried material (Formula LS (2%)) is passed through a # 20 mesh screen to achieve a useable yield of 95.2% and 1.3% oversized granules. Rapidly dispersing microgranules comprising Starch 1500 (at 1.0%: Formula LS - 1%; at 1.5%: Formula LS -1.5%; at 2.5%: Formula LS - 2.5%; at 3.0%: Formula LS - 3%) are also performed.

The particle size distributions that are obtained in each of the six granulations containing pre-gelatinized starch (PG starch) are measured using a Sonic shifter while the bulk and tap density values are also determined. From these values, percent compressibility values are calculated. Table 6 and FIG. 2 present the particle size distribution data for the 6 RD microgranule batches [5 batches of mannitol/L-HPC/Starch 1500 (at 1.0, 1.5, 2.0, 2.5, or 3.0%) and one batch of mannitol/crospovidone/Starch 1500 (2.5%)] in comparison to that of the RD microgranules, PE375 (Mannitol/crospovidone; no multi-functional additive, Pregelatinized Starch 1500), manufactured at industrial scale in accordance with US Patent 20050232988.

**Table 6: RD Microgranules - Granule Properties**

| **Microgranules Lot# (Disintegrant)** | **% PG Starch** | **Bulk Density (g/cc)** | **Tap Density (g/cc)** | **% Compressibility** |
|---|---|---|---|---|
| PE375 (Crospovidone) | none | 0.58 | 0.77 | 24.68 |
| Formula LS - 1% | 1.0 | 0.46 | 0.55 | 16.36 |
| Formula LS - 1.5% | 1.5 | 0.42 | 0.53 | 20.75 |
| Formula LS - 2% | 2.0 | 0.40 | 0.50 | 20.00 |
| Formula LS - 2.5% | 2.5 | 0.42 | 0.52 | 19.23 |
| Formula LS - 3% | 3.0 | 0.41 | 0.50 | 18.00 |
| Formula CS - 2% | 2.0 | 0.42 | 0.53 | 20.75 |

### Example 4.B: Orally Disintegrating Tablets

Low-substituted HPC (5 wt.%), microcrystalline cellulose (Avicel PH101 at 10%), sucralose (0.4%), and strawberry flavor (0.6%) are mixed in a polyethylene bag and passed through 40 mesh screen. The screened material is blended with the required amounts of acetaminophen microcapsules (38% by weight of lot at 10% EC-100 Coating), 46% by weight of rapidly dispersing granules comprising with pregelatinized starch as the granulation additive (Formula LS - 1.0%, Formula LS - 1.5%, Formula LS 2.0%, Formula LS - 2.5%, Formula LS - 3.0%), or rapidly dispersing microgranules without the multi-functional additive (PE375) in a 0.25 cu-ft V-blender for 10 min and compressed into 250 mg Acetaminophen ODTs weighing 700 mg using the Hata tablet press - Matsui ExLub system and 13 mm round, flat radius edge tooling at a compression force of 12 to 18 kN. All the tableting runs are smooth with no material flow related issues. The hardness and friability values that are observed at comparable compression forces for different ODT formulations are within narrow ranges (see FIG. 3 and FIG. 4 for different ODT formulations).

### Example 5.A: RD Microgranules containing L-HPC/Starch in Fluid Air FA 300

Mannitol 25 with a median particle size of about 15 µm (148.8 kg) and low-substituted hydroxypropylcellulose (8.0 kg of L-HPC) are co-milled by passing the mixture through a Quadro Comil® mill (0.032" = ∼ 104 µm screen and 0.275" spacer) rotating at 60 Hz or 1,446 rpm. Starch 1500® (3.2 kg of pre-gelatinized starch from Colorcon) with multi-functionality is slowly added to 156.8 kg of purified water USP in a stainless steel container, with agitation at 750±25 rpm, until dissolved. Fluid Air FA 300 granulator equipped with a top spray granulator bowl equipped with a product support 200 mesh stainless steel screen and a top spray gun with 3 heads (three 2.16 mm nozzles) and 3 peristaltic pumps is preheated while empty to reduce the amount of material sticking to the walls of the unit, if any. The pre-blended mixture of mannitol and L-HPC is charged into the pre-heated product bowl. The aqueous Starch 1500 solution described above is sprayed onto the blend and granulated at the following processing parameters - inlet air temperature: 100°C; air volume: 700-900 scfm; spray rate: 550 g/min (ramped up to 775 (Formula 2) or 1000 (Formula 3) g/min); atomization pressure: 4.0 bar; product temperature: 30-32°C. After spraying, the wet granules are dried to reduce the moisture in the granulation to below 2.0% at inlet temperature: 100°C; inlet air volume: 700 scfm; and end product temperature: 48°C. The dried granules are passed through a 20 mesh market grade screen using a Kason 30" sifter into fiber drums double lined with one inner anti-static polyethylene bags. The useable yield varied from 83% to 98% of the theoretical batch size. Bulk density: 0.47 g/cc and tap density: 0.63 g/cc. Three replicate batches (each 160 kg) of RD microgranules are also prepared at the same Starch 1500 content, but using varying amounts of water, as described above. The oversized granules may be milled if > 2% by weight. The process produces RD microgranules with very uniform particle size distributions and very high yields ranging from 95% to 99%, with less than 1% of oversized material.

### Example 5.B: RD Microgranules containing L-HPC/Starch in Glatt GPCG 120

Starch 1500® (3.2 kg) is slowly dissolved in 100 kg of purified water USP in a stainless steel container as described in Example 5.A above. The blend of Mannitol 25 (148.8 kg) and low-substituted hydroxypropylcellulose (8.0 kg of L-HPC) is milled by passing the mixture through the Comil® screen/spacer and granulated in the preheated Glatt GPCG 120 by spraying the aqueous Starch 1500 solution as disclosed in Example 5.A above at the following processing parameters - inlet air temperature: 100°C; air volume: 2500 scfm; spray rate:2000 g/min; atomization pressure: 3.0 bar; product temperature: 30-32°C. After spraying, the wet granules are dried to reduce the moisture in the granulation to below 2.0% at inlet temperature: 100°C; inlet air volume: 1,500 scfm; and end product temperature: 48°C. Four replicate batches (each 160 kg) of RD microgranules at the same Starch 1500 content and one batch (Formula E) at Starch 1500 content of 2.5% are also prepared as described above. The useable yield varied from 91 to 96% of the theoretical batch size. The particle size, bulk/tap density measurements are performed to determine median particle size and compressibility for the RD microgranule batches of Example 5.A and 5.B are presented in FIG. 5 and 6, respectively.

### Example 5.C: Acetaminophen ODTs containing Mannitol/L-HPC/Starch Microgranules

Low-substituted HPC (5 wt.%), microcrystalline cellulose (Avicel PH101 at 10%), sucralose (0.4%), and strawberry flavor (0.6%) are blended in a 0.5 cu-ft V-blender for 10 min and passed through 40 mesh screen. The screened material is blended with the required amounts of acetaminophen microcapsules (38% by weight of lot at 10% EC-100 Coating), 46% by weight of rapidly dispersing granules comprising with pre-gelatinized starch as the granulation additive - Ex. 5.B at 2%, or 2.5%, or rapidly dispersing microgranules without a binder (PE375, mannitl/crospovidone; no multi-functional additive) in a 2 cu-ft V-blender for 10 min and compressed into 250 mg Acetaminophen ODTs weighing 700 mg using the Hata tablet press - Matsui ExLub system and 13 mm round, flat radius edge tooling at a compression force of 12 to 18 kN.

Compression blend batches are compressed on a Hata Tablet Press equipped with an external lubricating system, Matsui Exlub System. The starting operating parameters are varied as needed to maintain tablet weight, hardness, thickness and friability within commercial tolerances. The weight range for the tablets is typically maintained with + 4% of the target tablet weight. The ExLub system is started to ensure that the lubricant is spraying properly when the tablet press is running. The tableting parameters, such as fill depth (mm), pre-compression position (mm or kN) and main compression position (mm or kN) are adjusted on the press in order to produce 250 mg tablets that meet the anticipated specifications. Following successful set-up, the press is run in 'Automatic Mode' until completion of the compression run. During the run, tablets are sampled periodically to ensure that the tablets produced would meet the specifications. The tablet weight, hardness and thickness are measured on a sample of five tablets every 30 min. Every 60 min a sufficient sample is also taken for friability testing. All the tableting runs are expected to be smooth without requiring an adjustment of operating parameters to keep the tablet attributes within the specifications. No flow-related processing problems or scoring are observed during these tableting runs. In addition, the added multi-functional additive has not increased *in vitro* or oral disintegration time compared to ODTs prepared without the granulation additive.

### Example 5.D: Acetaminophen/Hydrocodone ODTs containing Mannitol/L-HPC/Starch

Sucralose (1.0%), artificial cherry flavor (1.15%), microcrystalline cellulose (10% of Avicel PH101), and croscarmellose sodium (3% of Ac-Di-Sol) are pre-blended in a V blender for 5 min to achieve homogeneity followed by passing the pre-blend through a Comil® screen/spacer running at 1446 rpm to deagglomerate. The taste-masked hydrocodone/ acetaminophen microparticles (18.6%) from Comparative Example 2.E, above, taste-masked acetaminophen microcrystals (17.1%) from Comparative Example 2.E, the pre-blend, and the rapidly dispersing microgranules (48.2%) are blended in a V-blender for 20 min followed by blending with pre-screened sodium stearyl fumarate (1.0%) for 5 min. The ODT tablets, 300-mg/10-mg having sufficiently high tensile strength and low friability to withstand attrition during packaging in HDPE bottles, storage, and transportation are manufactured by compressing the compression blend. These tablets are found to disintegrate within 30 seconds when tested by USP Disintegration Time method <701>.

### Example 6.A: RD Microgranules comprising Pearlitol 60/L-HPC / Starch or Klucel LF

Pregelatinized Starch 1500 (120 g equivalent to 2% based on the weight of the microgranule) is slowly added to purified water in a stainless steel container while continuously stirring to dissolve. D-mannitol with a median particle size of about 60 µm (5580 g of Pearlitol 60) and low-substituted hydroxypropylcellulose (300 g) are granulated by spraying the starch solution in a Glatt GPCG 5 as disclosed in Example 4.A, above. Rapidly dispersing microgranules comprising low viscosity hydroxypropylcellulose (90 g of Klucel LF) as the granulation additive is slowly added to purified water at 60°C in a stainless steel container equipped with a heating jacket while continuously stirring to dissolve. D-mannitol with a median particle size of about 60 µm (5610 g of Pearlitol 60) and low-substituted hydroxypropylcellulose (300 g) are granulated by spraying the Klucel solution in a Glatt GPCG 5 as disclosed in Example 4.A, above.

### Example 6.B: RD Microgranules comprising Pearlitol 35/L-HPC/Starch

D-mannitol with a median particle size of about 35 µm (5580 g of Pearlitol 35) and low-substituted hydroxypropylcellulose (300 g) are granulated by spraying the starch solution (120 g Pregelatinzed Starch 1500) in a Glatt GPCG 5 as disclosed in Example 4.A, above.

### Comparative Example 6.C: CR Melperone Microparticle

Melperone hydrochloride ODT CR tablets are prepared by following the disclosures of United States Patent Application Ser. No. 12/639,496. Melperone hydrochloride (15.0 kg) is slowly added to a 50/50 mixture of acetone and water (50 kg each) with stirring until dissolved. The above melperone solution is sprayed onto 45-60 mesh sugar spheres (43.8 kg) in a Glatt GPCG 120 equipped with an 18" bottom spray Wurster 18" column, 3.0 mm nozzle port with HS Collar and bottom inner "G" and outer "C" distribution plate at a product temperature of 32°C (range: 29-36°C). Following drug layering, a seal coat of Klucel® LF at 2% by weight is applied, dried in the Glatt unit for 5 min to drive off residual solvents (including moisture), and sieved through 35 mesh (500 µm) screen to discard doubles, if any. Dibasic sodium phosphate (6.2 kg) is slowly added to 124 kg of purified water while stirring. Melperone hydrochloride IR beads (52.6 kg) are coated with the aqueous alkaline buffer solution at a product temperature: 53°C (range: 49-60°C). Following the buffer layering, a protective seal coat of Opadry Clear for a weight gain of about 2% at a product temperature of 50°C.

Ethylcellulose (13.9 kg) is slowly added to 85/15 acetone/water mixture, with stirring, until dissolved. Then dibutyl sebacate (1.1 kg) is slowly added to the polymer solution, and stirred for 30 min. The buffer-coated melperone IR beads (34.0 kg) are coated with the above SR coating solution (7% solids) in the Glatt at a product temperature: 33°C (range: 29-40°C). Following rinsing with acetone, the SR-coated beads are then sprayed with a seal-coat solution (Klucel® LF; 7.5% solids), dried in the Glatt unit for 5 min to drive off residual solvents (including moisture), and then sieved to discard oversized and fines, if any.

### Example 6.D: ODT CR Melperone containing Mannitol/L-HPC/Starch Microparticles

Crospovidone (5 wt.%), microcrystalline cellulose (Avicel PH101 at 10%), sucralose (0.4%), and peppermint flavor (1.0%) are blended in a 0.5 cu-ft V-blender for 10 min and passed through 40 mesh screen. The screened material is blended with the required amounts of melperone SR beads (36% by weight) from step 6B, 47.4% by weight of rapidly dispersing granules from Example 6 in a V-blender for 10 min and compressed into 50 mg melperone HCl ODT CR tablets weighing 1000 mg at a compression force of 12 to 18 kN.

## Claims

1. Pharmaceutically acceptable, rapidly dispersing microgranules having a median particle size in the range of 100 µm to 300 µm and comprising at least one sugar alcohol, saccharide, or a mixture thereof, at least one super disintegrant selected from the group consisting of crospovidone, croscarmellose sodium, sodium starch glycolate, low-substituted hydroxypropylcellulose and mixtures thereof, and pregelatinized starch in an amount of 0.5-3.0% by weight, wherein the rapidly dispersing microgranules do not comprise a pharmaceutically active agent.

2. The rapidly dispersing microgranules of claim 1 wherein the at least one sugar alcohol, saccharide, or a mixture thereof, at least one super disintegrant, and pregelatinized starch are present at a ratio of 88-96 (sugar alcohol): 1-10 (disintegrant): 1-3 (pregelatinized starch).

3. The rapidly dispersing microgranules of claim 1 wherein the sugar alcohol is selected from the group consisting of mannitol, xylitol and mixture thereof, and/or the saccharide is selected from the group consisting of lactose, sucrose, fructose, and mixture thereof.

4. The rapidly dispersing microgranules of claim 1 wherein the sugar alcohol is mannitol having a median particle size of 60 µm, and/or said disintegrant is low substituted hydroxypropylcellulose.

5. The rapidly dispersing microgranules of claim 1 wherein the sugar alcohol is mannitol having a median particle size of 15 to 30 µm, and/or said super disintegrant is low substituted hydroxypropylcellulose.

6. A pharmaceutical dosage form, which is an orally disintegrating tablet, comprising:
a) the rapidly dispersing microgranules of claim 1; and
b) a therapeutically effective amount of at least one active pharmaceutical ingredient.

7. The pharmaceutical dosage form of claim 6 wherein the active pharmaceutical ingredient further comprises one or more coatings of one or more functional polymers to impart taste-masking, controlled release characteristics, or a combination thereof, and optionally a pharmaceutically acceptable excipient.

8. The pharmaceutical dosage form of claim 7 wherein the active pharmaceutical ingredient is in the form of taste-masked microparticles having a median particle size in the range of 100 to 400 µm, and the orally disintegrating tablet dosage form rapidly disintegrates on contact with saliva in the buccal cavity of a mammal creating a smooth, non-gritty, and easy-to-swallow suspension containing the taste-masked drug microparticles, which provide a dissolution profile similar to that of the reference listed drug in order to be bioequivalent.

9. The pharmaceutical dosage form of claim 7 wherein the active pharmaceutical ingredient is in in the form of microparticles which are imparted with taste-masking, controlled release characteristics, or a combination thereof, and have a median particle size in the range of 100 to 400 µm, and the orally disintegrating tablet dosage form rapidly disintegrates on contact with saliva in the buccal cavity of a mammal creating a smooth, non-gritty, and easy-to-swallow suspension of the drug microparticles with taste-masking, controlled release characteristics, or a combination thereof, which provide a plasma concentration-time profile that is suitable for a once-a-day or twice-a-day dosing regimen.

10. The pharmaceutical dosage form of claim 6 or 7 wherein the orally disintegrating tablet dosage form is formed by compressing the ingredients of the orally disintegrating tablet composition on a rotary tablet press to achieve sufficiently high tablet hardness and low friability to withstand attrition during packaging in blisters or bottles, storage, transportation for commercial distribution and end use, wherein the orally disintegrating tablet dosage form is compressed on a rotary tablet press equipped with an external lubrication device to lubricate material contacting punch surfaces and die wall prior to each compression using a lubricant selected from the group consisting of magnesium stearate, stearic acid, calcium stearate, zinc stearate, sodium stearyl fumarate, and glyceryl behenate, or the ingredients of the orally disintegrating tablet dosage form are compressed after internally lubricating the ingredients with a lubricant selected from the group consisting of magnesium stearate, stearic acid, calcium stearate, zinc stearate, sodium stearyl fumarate, glyceryl behenate.

11. The pharmaceutical dosage form of claim 6 or 7 wherein the orally disintegrating tablet dosage form disintegrates within 30 seconds when tested for disintegration time by the United States Pharmacopeia method <701>.

12. The pharmaceutical dosage form of claim 6 or 7 wherein the therapeutic agent is selected from the group consisting of drugs for central nervous system, antidepressants, antiemetics, cardiovascular agents, antihypnotics / antianxiolytics, sedatives, antiepileptics, analgesics / antipyretic agents, rheumatoid arthritis, antimigraine drugs, opioids, drugs for Parkinson's disease, antipsychotic agents, antiplatelet drugs, skeletal muscle relaxants, anti-Alzheimer drugs, antispasmodic agents, proton pump inhibitors, histamine H2 antagonists, gastrointestinal disorders aminosalicylates, metronidazole, corticosteroids, antidiabetics, antiallergics, and antibiotic agents.

13. The pharmaceutical dosage form of claim 12 further comprising at least one pharmaceutically acceptable excipient selected from a flavorant, sweetener, colorant, compression aid, or additional disintegrant and wherein the composition is compressed into an orally disintegrating tablet using a rotary tablet press with internal or external lubrication, and the tablet disintegrates within 30 seconds when tested for disintegration time by the United States Pharmacopeia method <701>.

14. A method of manufacturing an orally disintegrating tablet comprising the following steps:
a. preparing active pharmaceutical ingredient microparticles,
b. coating the drug microparticles with one or more functional polymers to impart taste-masking and/or controlled release characteristics,
c. preparing a powder mixture comprising polymer coated drug microparticles having a median particle size in the range of 100 to 400 µm from step (b), free flowing, rapidly dispersing micro granules having a median particle size in the range of 100 to 300 µm of claim 1 or 2, and other optional pharmaceutically acceptable excipients selected from a flavorant, sweetener, colorant, compression aid, and additional disintegrant;
d. compressing the powder mixture on a rotary tablet press using internal or external lubrication, wherein the orally disintegrating tablet rapidly disintegrates on contact with saliva in the buccal cavity into a smooth, non-gritty, easy-to-swallow suspension containing polymer coated drug microparticles or drug microparticles taste-masked by granulating with a sugar alcohol, super disintegrant and optionally a flavorant or sweetener.

15. A method of manufacturing an orally disintegrating tablet comprising the following steps:
a. preparing active pharmaceutical ingredient microparticles,
b. optionally coating the drug microparticles with one or more functional polymers to impart taste-masking and/or controlled release characteristics,
c. preparing a powder mixture comprising polymer coated drug microparticles having a median particle size in the range of 100 to 400 µm from step (b), the micro granules of claim 1 or 2, and other optional pharmaceutically acceptable excipients selected from a flavorant, sweetener, colorant, compression aid, and additional disintegrant;
d. compressing the powder mixture on a rotary tablet press using internal or external lubrication, wherein the orally disintegrating tablet rapidly disintegrates on contact with saliva in the buccal cavity into a smooth, non-gritty, easy-to-swallow suspension containing polymer coated drug microparticles or drug microparticles taste-masked by granulating with a sugar alcohol, super disintegrant and optionally a flavorant or sweetener.

16. A tablet obtained by the method of claim 14 or 15 wherein the tablet is prepared by a method in which the powder mixture is compressed on a rotary tablet press without the addition of a lubricant to the blend, and the method includes a lubricating device to lubricate material contacting punch surfaces and die wall of the tablet press.

17. A tablet obtained by the method of claim 14 or 15 wherein the tablet is prepared by a method in which the powder mixture is compressed on a rotary tablet press after mixing with a lubricant selected from the group consisting of magnesium stearate, stearic acid, calcium stearate, zinc stearate, sodium stearyl fumarate, glyceryl behenate.

## Patentansprüche

1. Pharmazeutisch annehmbare, sich schnell auflösende Mikrogranulate, die eine mittlere Teilchengröße im Bereich von 100 µm bis 300 µm aufweisen und mindestens einen Zuckeralkohol, ein Saccharid oder ein Gemisch davon, mindestens ein Supersprengmittel, das aus der Gruppe bestehend aus Crospovidon, Croscarmellose-Natrium, Natriumstärkeglykolat, gering substituierter Hydroxypropylcellulose und Gemischen davon ausgewählt ist, und vorgelierte Stärke in einer Menge von 0,5-3,0 Gew.-% umfassen, wobei die sich schnell auflösenden Mikrogranulate kein pharmazeutisch aktives Mittel umfassen.

2. Sich schnell auflösende Mikrogranulate nach Anspruch 1, wobei der mindestens eine Zuckeralkohol, das Saccharid oder ein Gemisch davon, das mindestens eine Supersprengmittel und die vorgelierte Stärke in einem Verhältnis von 88-96 (Zuckeralkohol):1-10 (Sprengmittel):1-3 (vorgelierte Stärke) vorliegen.

3. Sich schnell auflösende Mikrogranulate nach Anspruch 1, wobei der Zuckeralkohol aus der Gruppe bestehend aus Mannit, Xylit und einem Gemisch davon ausgewählt ist und/oder das Saccharid aus der Gruppe bestehend aus Laktose, Saccharose, Fruktose und einem Gemisch davon ausgewählt ist.

4. Sich schnell auflösende Mikrogranulate nach Anspruch 1, wobei der Zuckeralkohol Mannit ist, das eine mittlere Teilchengröße von 60 µm aufweist, und/oder das Sprengmittel gering substituierte Hydroxypropylcellulose ist.

5. Sich schnell auflösende Mikrogranulate nach Anspruch 1, wobei der Zuckeralkohol Mannit ist, das eine mittlere Teilchengröße von 15 bis 30 µm aufweist, und/oder das Supersprengmittel gering substituierte Hydroxypropylcellulose ist.

6. Pharmazeutische Dosierungsform, die eine oral zerfallende Tablette ist, umfassend:
a) die sich schnell auflösenden Mikrogranulate nach Anspruch 1 und
b) eine therapeutisch wirksame Menge von mindestens einem aktiven pharmazeutischen Inhaltsstoff.

7. Pharmazeutische Dosierungsform nach Anspruch 6, wobei der aktive pharmazeutische Inhaltsstoff weiterhin einen oder mehrere Überzüge aus einem oder mehreren funktionellen Polymeren, um geschmacksmaskierende Charakteristika, Charakteristika einer kontrollierten Abgabe oder eine Kombination davon zu verleihen, und optional einen pharmazeutisch annehmbaren Hilfsstoff umfasst.

8. Pharmazeutische Dosierungsform nach Anspruch 7, wobei der aktive pharmazeutische Inhaltsstoff in der Form von geschmacksmaskierten Mikroteilchen ist, die eine mittlere Teilchengröße im Bereich von 100 bis 400 µm aufweisen, und die oral zerfallende Tablettendosierungsform bei Kontakt mit Speichel in der Mundhöhle eines Säugetiers schnell zerfällt, wodurch eine glatte, nicht körnige und leicht zu schluckende Suspension erzeugt wird, die die geschmacksmaskierten Wirkstoffmikroteilchen enthält, die ein Freisetzungsprofil bereitstellen, das dem des Originalwirkstoffpräparats ähnlich ist, um bioäquivalent zu sein.

9. Pharmazeutische Dosierungsform nach Anspruch 7, wobei der aktive pharmazeutische Inhaltsstoff in der Form von Mikroteilchen ist, denen geschmacksmaskierende Charakteristika, Charakteristika einer kontrollierten Abgabe oder eine Kombination davon verliehen werden, und die eine mittlere Teilchengröße im Bereich von 100 bis 400 µm aufweisen, und die oral zerfallende Tablettendosierungsform bei Kontakt mit Speichel in der Mundhöhle eines Säugetiers schnell zerfällt, wodurch eine glatte, nicht körnige und leicht zu schluckende Suspension der Wirkstoffmikroteilchen mit geschmacksmaskierenden Charakteristika, Charakteristika einer kontrollierten Abgabe oder einer Kombination davon erzeugt wird, die ein Plasmakonzentration-Zeit-Profil bereitstellen, das für einen einmal täglichen oder zweimal täglichen Dosierungsplan geeignet ist.

10. Pharmazeutische Dosierungsform nach Anspruch 6 oder 7, wobei die oral zerfallende Tablettendosierungsform durch Verpressen der Inhaltsstoffe der oral zerfallenden Tablettenzusammensetzung auf einer Rotationstablettenpresse geformt wird, um eine ausreichend hohe Tablettenhärte und eine ausreichend geringe Sprödigkeit zum Standhalten eines Abriebs während einer Verpackung in Blister oder Flaschen, einer Lagerung, einem Transport zum gewerblichen Vertrieb oder Endverwendung zu erzielen, wobei die oral zerfallende Tablettendosierungsform auf einer Rotationstablettenpresse verpresst wird, die mit einer externen Einfettvorrichtung ausgestattet ist, um materialberührende Stanzflächen und eine Düsenwand vor jedem Verpressen unter Verwendung eines Gleitmittels, das aus der Gruppe bestehend aus Magnesiumstearat, Stearinsäure, Calciumstearat, Zinkstearat, Natriumstearylfumarat und Glycerylbehenat ausgewählt ist, einzufetten, oder die Inhaltsstoffe der oral zerfallenden Tablettendosierungsform verpresst werden, nachdem die Inhaltsstoffe mit einem Gleitmittel, das aus der Gruppe bestehend aus Magnesiumstearat, Stearinsäure, Calciumstearat, Zinkstearat, Natriumstearylfumarat, Glycerylbehenat ausgewählt ist, intern eingefettet wurden.

11. Pharmazeutische Dosierungsform nach Anspruch 6 oder 7, wobei die oral zerfallende Tablettendosierungsform innerhalb von 30 Sekunden zerfällt, wenn sie mittels des United-States-Pharmacopeia-Verfahrens <701> auf die Zerfallszeit getestet wird.

12. Pharmazeutische Dosierungsform nach Anspruch 6 oder 7, wobei das Therapeutikum aus der Gruppe bestehend aus Wirkstoffen für das Zentralnervensystem, Antidepressiva, Antiemetika, Herz-Kreislauf-Mittel, Antihypnotika/Antianxiolytika, Sedativa, Antiepileptika, Analgetika/Antipyretika, für rheumatoide Arthritis, Wirkstoffen gegen Migräne, Opioiden, Wirkstoffen für Parkinson-Krankheit, Antipsychotika, Thrombozytenaggregationshemmern, Skelettmuskulatur-Relaxantien, Wirkstoffen gegen Alzheimer-Krankheit, Antispasmodika, Protonenpumpenhemmern, Histamin-H2-Antagonisten, Aminosalicylaten für Magen-Darm-Krankheiten, Metronidazol, Kortikosteroiden, Antidiabetika, Antiallergika und Antibiotika ausgewählt ist.

13. Pharmazeutische Dosierungsform nach Anspruch 12, weiterhin umfassend mindestens einen pharmazeutisch annehmbaren Hilfsstoff, der aus einem Geschmacksstoff, Süßstoff, Farbstoff, Verpresshilfsstoff oder zusätzlichen Sprengmittel ausgewählt ist, und wobei die Zusammensetzung zu einer oral zerfallenden Tablette unter Verwendung einer Rotationstablettenpresse mit internem oder externem Einfetten verpresst wird und die Tablette innerhalb von 30 Sekunden zerfällt, wenn sie mittels des United-States-Pharmacopeia-Verfahrens <701> auf die Zerfallszeit getestet wird.

14. Verfahren zur Fertigung einer oral zerfallenden Tablette, umfassend die folgenden Schritte:
a. Herstellen von aktiven pharmazeutischen Inhaltsstoffmikroteilchen,
b. Überziehen der Wirkstoffmikroteilchen mit einem oder mehreren funktionellen Polymeren, um geschmacksmaskierende Charakteristika und/oder Charakteristika einer kontrollierten Abgabe zu verleihen,
c. Herstellen eines Pulvergemischs, das polymerüberzogene Wirkstoffmikroteilchen, die eine mittlere Teilchengröße im Bereich von 100 bis 400 µm aufweisen, aus Schritt (b), rieselfähige, sich schnell auflösende Mikrogranulate, die eine mittlere Teilchengröße im Bereich von 100 bis 300 µm aufweisen, nach Anspruch 1 oder 2 und andere optionale pharmazeutisch annehmbare Hilfsstoffe, die aus einem Geschmacksstoff, Süßstoff, Farbstoff, Verpresshilfsstoff oder zusätzlichen Sprengmittel ausgewählt sind, umfasst;
d. Verpressen des Pulvergemischs auf einer Rotationstablettenpresse unter Verwendung von internem oder externem Einfetten, wobei die oral zerfallende Tablette bei Kontakt mit Speichel in der Mundhöhle schnell zu einer glatten, nicht körnigen, leicht zu schluckenden Suspension zerfällt, die polymerüberzogene Wirkstoffmikroteilchen oder Wirkstoffmikroteilchen, die durch Granulieren mit einem Zuckeralkohol, Supersprengmittel und optional einem Geschmacksstoff oder Süßstoff geschmacksmaskiert sind, enthält.

15. Verfahren zur Fertigung einer oral zerfallenden Tablette, umfassend die folgenden Schritte:
a. Herstellen von aktiven pharmazeutischen Inhaltsstoffmikroteilchen,
b. optionales Überziehen der Wirkstoffmikroteilchen mit einem oder mehreren funktionellen Polymeren, um geschmacksmaskierende Charakteristika und/oder Charakteristika einer kontrollierten Abgabe zu verleihen,
c. Herstellen eines Pulvergemischs, das polymerüberzogene Wirkstoffmikroteilchen, die eine mittlere Teilchengröße im Bereich von 100 bis 400 µm aufweisen, aus Schritt (b), die Mikrogranulate nach Anspruch 1 oder 2 und andere optionale pharmazeutisch annehmbare Hilfsstoffe, die aus einem Geschmacksstoff, Süßstoff, Farbstoff, Verpresshilfsstoff oder zusätzlichen Sprengmittel ausgewählt sind, umfasst;
d. Verpressen des Pulvergemischs auf einer Rotationstablettenpresse unter Verwendung von internem oder externem Einfetten, wobei die oral zerfallende Tablette bei Kontakt mit Speichel in der Mundhöhle schnell zu einer glatten, nicht körnigen, leicht zu schluckenden Suspension zerfällt, die polymerüberzogene Wirkstoffmikroteilchen oder Wirkstoffmikroteilchen, die durch Granulieren mit einem Zuckeralkohol, Supersprengmittel und optional einem Geschmacksstoff oder Süßstoff geschmacksmaskiert sind, enthält.

16. Tablette, die durch das Verfahren nach Anspruch 14 oder 15 erhalten wird, wobei die Tablette durch ein Verfahren hergestellt wird, in dem das Pulvergemisch auf einer Rotationstablettenpresse ohne die Zugabe eines Gleitmittels zu der Mischung verpresst wird, und das Verfahren eine Einfettvorrichtung beinhaltet, um materialberührende Stanzflächen und eine Düsenwand der Tablettenpresse einzufetten.

17. Tablette, die durch das Verfahren nach Anspruch 14 oder 15 erhalten wird, wobei die Tablette durch ein Verfahren hergestellt wird, in dem das Pulvergemisch auf einer Rotationstablettenpresse nach einem Mischen mit einem Gleitmittel, das aus der Gruppe bestehend aus Magnesiumstearat, Stearinsäure, Calciumstearat, Zinkstearat, Natriumstearylfumarat, Glycerylbehenat ausgewählt ist, verpresst wird.

## Revendications

1. Microgranules à dispersion rapide pharmaceutiquement acceptables ayant une taille médiane des particules dans la plage de 100 µm à 300 µm et comprenant au moins un alcool de sucre, un saccharide ou un mélange de ceux-ci, au moins un super-délitant choisi dans le groupe constitué par la crospovidone, la croscarmellose sodique, le glycolate d'amidon sodique, l'hydroxypropylcellulose à faible substitution et des mélanges de ceux-ci et de l'amidon prégélatinisé en une quantité de 0,5-3,0 % en poids, les microgranules à dispersion rapide ne comprenant pas un agent pharmaceutiquement actif.

2. Microgranules à dispersion rapide selon la revendication 1, l'au moins un alcool de sucre, l'au moins un saccharide ou un mélange de ceux-ci, l'au moins un super-délitant et l'amidon prégélatinisé étant présents en un rapport de 88-96 (alcool de sucre):1-10 (délitant):1-3 (amidon prégélatinisé).

3. Microgranules à dispersion rapide selon la revendication 1, l'alcool de sucre étant choisi dans le groupe constitué par le mannitol, le xylitol et un mélange de ceux-ci et/ou le saccharide étant choisi dans le groupe constitué par le lactose, le saccharose, le fructose et un mélange de ceux-ci.

4. Microgranules à dispersion rapide selon la revendication 1, l'alcool de sucre étant le mannitol ayant une taille médiane des particules de 60 µm et/ou ledit délitant étant l'hydroxypropylcellulose à faible substitution.

5. Microgranules à dispersion rapide selon la revendication 1, l'alcool de sucre étant le mannitol ayant une taille médiane des particules de 15 à 30 µm et/ou ledit super-délitant étant l'hydroxypropylcellulose à faible substitution.

6. Forme galénique pharmaceutique, qui est un comprimé se délitant oralement, comprenant :
a) les microgranules à dispersion rapide selon la revendication 1 ; et
b) une quantité thérapeutiquement efficace d'au moins un principe pharmaceutique actif.

7. Forme galénique pharmaceutique selon la revendication 6, le principe pharmaceutique actif comprenant en outre un ou plusieurs enrobages d'un ou plusieurs polymères fonctionnels pour conférer un masquage de goût, des caractéristiques de libération contrôlée ou une combinaison de ceux-ci, et éventuellement un excipient pharmaceutiquement acceptable.

8. Forme galénique pharmaceutique selon la revendication 7, le principe pharmaceutique actif étant sous la forme de microparticules à goût masqué ayant une taille médiane des particules dans la plage de 100 à 400 µm et la forme galénique de comprimé se délitant oralement se délitant rapidement au contact avec de la salive dans la cavité buccale d'un mammifère ce qui crée une suspension onctueuse, non granuleuse et facile à avaler contenant les microparticules de médicament à goût masqué, qui fournissent un profil de dissolution similaire à celui du médicament de référence afin d'être bioéquivalentes.

9. Forme galénique pharmaceutique selon la revendication 7, le principe pharmaceutique actif étant sous la forme de microparticules auxquelles sont conférées un masquage de goût, des caractéristiques de libération contrôlée ou une combinaison de ceux-ci, et qui ont une taille médiane des particules dans la plage de 100 à 400 µm, et la forme galénique de comprimé se délitant oralement se délitant rapidement au contact avec de la salive dans la cavité buccale d'un mammifère ce qui crée une suspension onctueuse, non granuleuse et facile à avaler des microparticules de médicament à masquage de goût, caractéristiques de libération contrôlée ou une combinaison de ceux-ci, qui fournissent un profil de concentration plasmatique en fonction du temps qui est approprié pour un régime d'administration une fois par jour ou deux fois par jour.

10. Forme galénique pharmaceutique selon la revendication 6 ou 7, la forme galénique de comprimé se délitant oralement étant formée par compression des ingrédients de la composition de comprimé se délitant oralement sur une presse à comprimés rotative pour parvenir à une dureté de comprimé suffisamment élevée et une friabilité suffisamment faible pour résister à l'attrition pendant l'emballage dans des plaquettes alvéolaires ou des flacons, le stockage, le transport pour la distribution commerciale et l'utilisation finale, la forme galénique de comprimé se délitant oralement étant comprimée sur une presse à comprimés rotative pourvue d'une dispositif de lubrification externe pour lubrifier la matière en contact avec les surfaces de poinçon et la paroi de matrice avant chaque compression à l'aide d'un lubrifiant choisi dans le groupe constitué par le stéarate de magnésium, l'acide stéarique, le stéarate de calcium, le stéarate de zinc, le fumarate de sodium et de stéaryle et le béhénate de glycéryle, ou les ingrédients de la forme galénique de comprimé se délitant oralement étant comprimés après lubrification interne des ingrédients avec un lubrifiant choisi dans le groupe constitué par le stéarate de magnésium, l'acide stéarique, le stéarate de calcium, le stéarate de zinc, le fumarate de sodium et de stéaryle, le béhénate de glycéryle.

11. Forme galénique pharmaceutique selon la revendication 6 ou 7, la forme galénique de comprimé se délitant oralement se délitant en moins de 30 secondes lorsqu'elle est testée en ce qui concerne le temps de délitement par la méthode <701> de la pharmacopée des États-Unis.

12. Forme galénique pharmaceutique selon la revendication 6 ou 7, l'agent thérapeutique étant choisi dans le groupe constitué par les médicaments pour le système nerveux central, les antidépresseurs, les antiémétiques, les agents cardiovasculaires, les antihypnotiques/antianxiolytiques, les sédatifs, les antiépileptiques, les analgésiques,/agents antipyrétiques, la polyarthrite rhumatoïde, les médicaments antimigraineux, les opioïdes, les médicaments pour la maladie de Parkinson, les agents antipsychotiques, les médicaments antiagrégants plaquettaires, les relaxants des muscles squelettiques, les médicaments anti-Alzheimer, les agents antispasmodiques, les inhibiteurs de la pompe à protons, les antagonistes de l'histamine H2, les aminosalicylates pour les troubles gastro-intestinaux, le métronidazole, les corticostéroïdes, les antidiabétiques, les antiallergiques et les agents antibiotiques.

13. Forme galénique pharmaceutique selon la revendication 12 comprenant en outre au moins un excipient pharmaceutiquement acceptable choisi entre un agent aromatisant, un édulcorant, un colorant, un adjuvant de compression et un délitant supplémentaire et la composition étant comprimée en un comprimé se délitant oralement à l'aide d'une presse à comprimés rotative à lubrification interne ou externe et le comprimé se délitant en moins de 30 secondes lorsqu'il est testé en ce qui concerne le temps de délitement par la méthode <701> de la pharmacopée des États-Unis.

14. Procédé de fabrication d'un comprimé se délitant oralement comprenant les étapes suivantes :
a. la préparation de microparticules de principe pharmaceutique actif,
b. l'enrobage des microparticules de médicament avec un ou plusieurs polymères fonctionnels pour conférer un masquage de goût et/ou des caractéristiques de libération contrôlée,
c. la préparation d'un mélange de poudres comprenant des microparticules de médicament enrobées de polymère ayant une taille médiane des particules dans la plage de 100 à 400 µm provenant de l'étape (b), des microgranules à dispersion rapide coulants ayant une taille médiane des particules dans la plage de 100 à 300 µm selon la revendication 1 ou 2 et d'autres excipients pharmaceutiquement acceptables facultatifs choisis entre un agent aromatisant, un édulcorant, un colorant, un adjuvant de compression et un délitant supplémentaire ;
d. la compression du mélange de poudres sur une presse à comprimés rotative à l'aide d'une lubrification interne ou externe, le comprimé se délitant oralement se délitant rapidement au contact avec de la salive dans la cavité buccale en une suspension onctueuse, non granuleuse, facile à avaler contenant des microparticules de médicament enrobées de polymère ou des microparticules de médicament à goût masqué par granulation avec un alcool de sucre, un super-délitant et éventuellement un agent aromatisant ou un édulcorant.

15. Procédé de fabrication d'un comprimé se délitant oralement comprenant les étapes suivantes :
a. la préparation de microparticules de principe pharmaceutique actif,
b. éventuellement l'enrobage des microparticules de médicament avec un ou plusieurs polymères fonctionnels pour conférer un masquage de goût et/ou des caractéristiques de libération contrôlée,
c. la préparation d'un mélange de poudres comprenant des microparticules de médicament enrobées de polymère ayant une taille médiane des particules dans la plage de 100 à 400 µm provenant de l'étape (b), des microgranules selon la revendication 1 ou 2 et d'autres excipients pharmaceutiquement acceptables facultatifs choisis entre un agent aromatisant, un édulcorant, un colorant, un adjuvant de compression et un délitant supplémentaire ;
d. la compression du mélange de poudres sur une presse à comprimés rotative à l'aide d'une lubrification interne ou externe, le comprimé se délitant oralement se délitant rapidement au contact avec de la salive dans la cavité buccale en une suspension onctueuse, non granuleuse, facile à avaler contenant des microparticules de médicament enrobées de polymère ou des microparticules de médicament à goût masqué par granulation avec un alcool de sucre, un super-délitant et éventuellement un agent aromatisant ou un édulcorant.

16. Comprimé obtenu par le procédé selon la revendication 14 ou 15, le comprimé étant préparé par un procédé dans lequel le mélange de poudres est comprimé sur une presse à comprimés rotative sans l'ajout d'un lubrifiant au mélange, et le procédé comprenant un dispositif de lubrification pour lubrifier la matière en contact avec les surfaces de poinçon et la paroi de matrice de la presse à comprimés.

17. Comprimé obtenu par le procédé selon la revendication 14 ou 15, le comprimé étant préparé par un procédé dans lequel le mélange de poudres est comprimé sur une presse à comprimés rotative après mélange avec un lubrifiant choisi dans le groupe constitué par le stéarate de magnésium, l'acide stéarique, le stéarate de calcium, le stéarate de zinc, le fumarate de sodium et de stéaryle, le béhénate de glycéryle.
